(19) **Europäisches Patentamt / European Patent Office / Office européen des brevets**

(11) **EP 4 362 990 B1**

(12) # EUROPEAN PATENT SPECIFICATION

(45) Date of publication and mention of the grant of the patent:
**29.07.2026  Bulletin 2026/31**

(21) Application number: **22832269.9**

(22) Date of filing: **25.05.2022**

(51) International Patent Classification (IPC):
**A61L 2/10** *(2026.01)*   **G02B 5/26** *(2006.01)*
**A61L 2/24** *(2006.01)*   **G02B 5/08** *(2006.01)*
**G02B 5/00** *(2006.01)*   **G02B 5/02** *(2006.01)*
**G02B 19/00** *(2006.01)*

(52) Cooperative Patent Classification (CPC):
**A61L 2/10; A61L 2/24; G02B 5/0825;**
A61L 2103/15; A61L 2202/14; A61L 2202/15;
A61L 2209/111; A61L 2209/12; A61L 2209/134;
G02B 5/003; G02B 5/02; G02B 5/0891;
G02B 19/0019; Y02A 50/30

(86) International application number:
**PCT/IB2022/054926**

(87) International publication number:
**WO 2023/275633 (05.01.2023 Gazette 2023/01)**

(54) **DEVICES INCLUDING A MULTILAYER ARTICLE HAVING AN ABSORBENT LAYER AND AN ULTRAVIOLET MIRROR, SYSTEMS, AND METHODS OF DISINFECTING**

VORRICHTUNGEN MIT EINEM MEHRSCHICHTIGEN ARTIKEL MIT EINER ABSORBIERENDEN SCHICHT UND EINEM UV-SPIEGEL, SYSTEME UND DESINFEKTIONSVERFAHREN

DISPOSITIFS COMPRENANT UN ARTICLE MULTICOUCHE AYANT UNE COUCHE ABSORBANTE ET UN MIROIR À ULTRAVIOLET, SYSTÈMES, ET PROCÉDÉS DE DÉSINFECTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **29.06.2021   US 202163216246 P**

(43) Date of publication of application:
**08.05.2024   Bulletin 2024/19**

(73) Proprietor: **3M Innovative Properties Company**
**Saint Paul, Minnesota 55133-3427 (US)**

(72) Inventors:
• **HEBRINK, Timothy J.**
  **Saint Paul, Minnesota 55133-3427 (US)**

• **WHEATLEY, John A.**
  **Saint Paul, Minnesota 55133-3427 (US)**
• **ACHARYA, Bharat R.**
  **Saint Paul, Minnesota 55133-3427 (US)**
• **LIU, Tao**
  **Saint Paul, Minnesota 55133-3427 (US)**

(74) Representative: **Mathys & Squire**
**Theatinerstraße 8**
**80333 München (DE)**

(56) References cited:
WO-A1-2016/196904    WO-A1-2016/196904
WO-A1-2017/176083    WO-A1-2017/176083
WO-A1-2020/070589    WO-A1-2020/070589
JP-A- 2018 175 506    US-A1- 2017 095 585

**Description**

<u>Field</u>

The present disclosure generally relates to the use of selected wavelengths of ultraviolet (UV) light.

<u>Background</u>

Ultraviolet (UV) light is useful, for example, for initiating free radical reaction chemistries used in coatings, adhesives, and polymeric materials. Ultraviolet light is also useful, for example, for disinfecting surfaces, filters, bandages, membranes, articles, air, and liquids (e.g., water). Examples where UVC (i.e., ultraviolet C includes wavelengths in a range from 100 nanometers to 280 nanometers) disinfection could be applied include medical offices and supplies, airplane restrooms, hospital rooms and surgical equipment, schools, air and water purification, and consumer applications (e.g., toothbrush and cell phone disinfection). Prevention of infection and spread of disease, especially in high-risk environments and populations, has become increasingly more critical as pathogens mutate and develop antibiotic resistance. The availability and speed of global human travel elevates risks of rapidly developed epidemics/pandemics. Air and water disinfection is paramount to human health and preventing infectious disease. Benefits of UVC disinfection include touch-free application, and the mechanical disruption of cells at non-gene specific targets is unlikely to be overcome by pathogens via mutation to develop resistance. Surfaces being disinfected with ultraviolet light other than metal, ceramic, or glass surfaces will need protection from ultra-violet light. UVC irradiation can be applied to effectively inactivate or kill prokaryotic and eukaryotic microorganisms alike, including bacteria, viruses, fungi and molds. Bacterial strains with developed resistance to one or more antibiotics are also susceptible to UVC light. Some examples of pathogens of heightened interest include hospital acquired infections (e.g., C. diff, E. coli, MRSA, Klebsiella, influenza, mycobacteria, and enterobacteria), water and soil borne infections (e.g., giardia, legionella, and campylobacter) and airborne infections (e.g., influenza, pneumonia, and tuberculosis).

UV light, however, can also be harmful to people and animals in varying degrees. For example, UV light sources that emit 400 nm to 500 nm wavelength light may cause long term damage to the eyes. WO 2020/070589 A1 discloses a device comprising a UVC impermeable housing with a UVC light source within, an absorbent layer and a an ultraviolet mirror below the absorbent layer.

<u>Summary</u>

In a first aspect, a device is provided. The device includes a) a housing that is substantially impermeable to ultraviolet radiation having a wavelength of from 280 nm to 400 nm, and at least one window defined in the housing; b) a broadband UVC light source positioned within the housing or within an optional enclosure adjacent to the housing; and c) a multilayer article positioned within the housing. The broadband UVC light source is configured to direct light at the ultraviolet mirror of the multilayer article. The multilayer article includes i) an absorbent layer that absorbs, scatters, or both, at least 50, 60, 70, 80, 90, or 95 percent of incident ultraviolet light having a wavelength between at least 230 nanometers and 400 nanometers. The absorbent layer has a major surface. The multilayer article further comprises ii) an ultraviolet mirror adjacent to the major surface of the absorbent layer, wherein the ultraviolet mirror is comprised of at least a plurality of alternating first and second optical layers collectively reflecting at an incident light angle of at least one of 0°, 15°, 30°, 45°, 60°, or 75°, at least 50, 60, 70, 80, 90, or 95 percent of incident ultraviolet light in a wavelength range from 195 nanometers or 200 nm, to 230 nanometers, 235 nm, or 240 nm, and collectively transmitting at an incident light angle of at least one of 0°, 15°, 30°, 45°, 60°, or 75°, at least 50, 60, 70, 80, 90, or 95 percent of incident ultraviolet light in a wavelength range from greater than 230 nanometers, greater than 235 nm, or greater than 240 nm, to 400 nanometers.

In a second aspect, a method of disinfecting at least one material is provided. The method includes obtaining a device according to the first aspect and directing ultraviolet radiation emitted by the broadband UVC light source through the window; and exposing the at least one material to the ultraviolet radiation passing through the window for a time sufficient to achieve a desired degree of disinfection of the at least one material. Optionally, exposing until achievement of a log 2, log 3, log 4, or greater reduction in an amount of at least one microorganism present on or in the at least one material, as compared to an amount of the at least one microorganism present prior to exposing the at least one material to the ultraviolet radiation passing through the window. The method is useful, for example, for disinfecting materials such as surfaces (e.g., walls, floors, furniture), medical instruments, hygiene articles, air, liquids (e.g., water or beverages), filter media, food preparation devices, and porous membranes.

In a third aspect, a system is provided. The system includes a device according to the first aspect; one or more electronic sensors configured to generate data that is indicative of an operation of the device; and at least one computing device including one or more computer processors and a memory including instructions. When the instructions are executed by the one or more computer processors, the execution causes the one or more computer processors to: receive

**EP 4 362 990 B1**

the data that is indicative of the operation of the device; apply the data to a disinfection model stored in the memory, the disinfection model characterizing the activity of the device; and wirelessly send to the device feedback that is based on the disinfection model, based on the identification of device activity.

In a fourth aspect, a computing device is provided. The computing device includes one or more computer processors. The computing device further includes a memory including instructions that when executed by the one or more computer processors cause the one or more computer processors to: obtain usage data from a device of the first aspect, wherein the usage data comprises data indicative of operation of the device; apply the usage data to a disinfection model stored in the memory, the disinfection model characterizing the activity of the device; and wirelessly send to the device feedback that is based on the disinfection model, based on the identification of device activity.

Brief Description of the Drawings

FIG. 1A is a schematic cross-sectional view of a multilayer article preparable according to the present disclosure.

FIG. 1B is a schematic cross-sectional view of another multilayer article preparable according to the present disclosure.

FIG. 2 is a graph of reflection spectra of an ultraviolet mirror prepared in Preparative Example 1.

FIG. 3 is a schematic cross-sectional view of an exemplary device preparable according to the present disclosure.

FIG. 4A is a schematic cross-sectional view of another exemplary device preparable according to the present disclosure.

FIG. 4B is a schematic cross-sectional view of an additional exemplary device preparable according to the present disclosure.

FIG. 5A is a schematic cross-sectional view of the modeled exemplary device of Example 1.

FIG. 5B is a schematic cross-sectional view of the modeled exemplary device of Example 1, including a representation of how the light rays might travel from inside to outside of the device cavity.

FIG. 5C is a graph of simulated system efficiency versus ultraviolet mirror reflectivity of the modeled exemplary device of Example 1.

FIG. 6A is a schematic cross-sectional view of the modeled exemplary device of Example 2.

FIG. 6B is a schematic cross-sectional view of the modeled exemplary device of Example 2, including a representation of how the light rays might travel from inside to outside of the device cavity.

FIG. 6C is a graph of simulated illuminance of the modeled exemplary device of Example 2 at 222 nanometers.

FIG. 6D is a graph of simulated illuminance of the modeled exemplary device of Example 2 at 254 nanometers.

FIG. 7A is a schematic cross-sectional view of the modeled exemplary device of Example 3.

FIG. 7B is a schematic cross-sectional view of the modeled exemplary device of Example 3, including a representation of how the light rays might travel from inside to outside of the device cavity.

FIG. 7C is a graph of simulated illuminance of the modeled exemplary device of Example 3 at 222 nanometers.

FIG. 7D is a graph of simulated illuminance of the modeled exemplary device of Example 3 at 254 nanometers.

FIG. 8A is a schematic cross-sectional view of the modeled exemplary device of Example 4.

FIG. 8B is a schematic cross-sectional view of the modeled exemplary device of Example 4, including a representation

3

of how the light rays might travel from inside to outside of the device cavity.

FIG. 8C is a graph of simulated illuminance of the modeled exemplary device of Example 4 at 222 nanometers.

FIG. 8D is a graph of simulated illuminance of the modeled exemplary device of Example 4 at 254 nanometers.

FIG. 9 a flow chart of an exemplary method according to the present disclosure.

FIG. 10 is a block diagram illustrating an exemplary system in which devices having communication capabilities are utilized and managed, according to aspects of this disclosure.

FIG. 11 is a block diagram illustrating an operating perspective of one example implementation of the equipment management system shown in FIG. 10.

Detailed Description

Glossary

As used herein, "fluoropolymer" refers to any organic polymer containing fluorine.

As used herein, "incident" with respect to light refers to the light falling on or striking a material.

As used herein, the term or prefix "micro" refers to at least one dimension defining a structure or shape being in a range from 1 micrometer to 1 millimeter. For example, a microstructure may have a height or a width that is in a range from 1 micrometer to 1 millimeter.

As used herein, the term or prefix "nano" refers to at least one dimension defining a structure or a shape being less than 1 micrometer. For example, a nano-structure may have at least one of a height or a width that is less than 1 micrometer.

As used herein, "radiation" refers to electromagnetic radiation unless otherwise specified.

As used herein, "absorption" refers to a material converting the energy of light radiation to internal energy.

As used herein, "absorb" with respect to wavelengths of light encompasses both absorption and scattering, as scattered light also eventually gets absorbed.

As used herein, "scattering" with respect to wavelengths of light refers to causing the light to depart from a straight path and travel in different directions with different intensities.

As used herein, "reflectance" is the measure of the proportion of light or other radiation striking a surface at normal incidence which is reflected off it. Reflectivity typically varies with wavelength and is reported as the percent of incident light that is reflected from a surface (0 percent - no reflected light, 100 - all light reflected. Reflectivity and reflectance are used interchangeably herein.

As used herein, "reflective" and "reflectivity" refer to the property of reflecting light or radiation, especially reflectance as measured independently of the thickness of a material.

As used herein, "average reflectance" refers to reflectance averaged over a specified wavelength range.

Absorbance can be measured with methods described in ASTM E903-12 "Standard Test Method for Solar Absorptance, Reflectance, and Transmittance of Materials Using Integrating Spheres". Absorbance measurements described herein were made by making transmission measurements as previously described and then calculating absorbance using Equation 1.

As used herein, the term "absorbance" with respect to a quantitative measurement refers to the base 10 logarithm of a ratio of incident radiant power to transmitted radiant power through a material. The ratio may be described as the radiant flux received by the material divided by the radiant flux transmitted by the material. Absorbance (A) may be calculated based on transmittance (T) according to Equation 1:

$$A = -\log_{10} T \qquad (1)$$

Emissivity can be measured using infrared imaging radiometers with methods described in ASTM E1933-14 (2018) "Standard Practice for Measuring and Compensating for Emissivity Using Infrared Imaging Radiometers."

The term "substantially" with reference to a property or characteristic means that the property or characteristic is exhibited to a greater extent than the opposite of that property or characteristic is exhibited. For example, a substrate that is "substantially" transparent refers to a substrate that transmits more radiation (e.g., visible light) than it fails to transmit (e.g., absorbs and reflects). Thus, a substrate that transmits more than 50% of the visible light incident upon its surface is substantially transparent, but a substrate that transmits 50% or less of the visible light incident upon its surface is not substantially transparent. Concomitantly, a substrate that is "substantially" impermeable to radiation of a certain wavelength range blocks (e.g., absorbs and reflects) more than 50% of those wavelengths of radiation, and optionally more than 60%, 70%, 80%, or more than 90% of those wavelengths of radiation.

*Devices*

**In** a first aspect, a device is provided. The device comprises:

a) a housing that is substantially impermeable to ultraviolet radiation having a wavelength of from 280 nm to 400 nm, and at least one window defined in the housing;

b) a broadband UVC light source positioned within the housing or within an optional enclosure adjacent to the housing; and

c) a multilayer article positioned within the housing, the multilayer article comprising:

i) an absorbent layer that absorbs, scatters, or both, at least 50, 60, 70, 80, 90, or 95 percent of incident ultraviolet light having a wavelength between at least 230 nanometers and 400 nanometers, the absorbent layer comprising a major surface; and

ii) an ultraviolet mirror adjacent to the major surface of the absorbent layer, wherein the ultraviolet mirror is comprised of at least a plurality of alternating first and second optical layers collectively reflecting at an incident light angle of at least one of 0°, 15°, 30°, 45°, 60°, or 75°, at least 50, 60, 70, 80, 90, or 95 percent of incident ultraviolet light in a wavelength range from 195 nanometers or 200 nm, to 230 nanometers, 235 nm, or 240 nm, and collectively transmitting at an incident light angle of at least one of 0°, 15°, 30°, 45°, 60°, or 75°, at least 50, 60, 70, 80, 90, or 95 percent of incident ultraviolet light in a wavelength range from greater than 230 nanometers, greater than 235 nm, or greater than 240 nm, to 400 nanometers, wherein the broadband UVC light source is configured to direct light at the ultraviolet mirror of the multilayer article.

It may alternatively be desired for the ultraviolet mirror to reflect ultraviolet light having a somewhat different wavelength range than 195 nm to 240 nm and to transmit ultraviolet light at a concomitantly different wavelength range than greater than 240 nm to 400 nm. For instance, in some embodiments, the ultraviolet mirror reflects ultraviolet light in a wavelength range of 195 nm or 200 nm to any of 230 nm, 235 nm, or 240 nm; such as from 195 nm to 230 nm, from 200 nm to 240 nm, or from 200 nm to 230 nm. In such embodiments, the ultraviolet mirror transmits ultraviolet light in a wavelength range greater than the upper limit of the wavelength range that is reflected, i.e., greater than 230 nm, greater than 235 nm, or greater than 240 nm. For each of these wavelengths / wavelength ranges, it is to be understood that the ultraviolet mirror is exposed to incident light angle of at least one of 0°, 15°, 30°, 45°, 60°, or 75°, and the optical layers collectively reflect at least 50, 60, 70, 80, 90, or 95 percent of the incident ultraviolet light in the specified wavelength range; and collectively transmits at an incident light angle of at least one of 0°, 15°, 30°, 45°, 60°, or 75°, at least 50, 60, 70, 80, 90, or 95 percent of incident ultraviolet light in the specified wavelength range.

It is to be understood that the percent of incident light absorbed refers to the amount absorbed integrated over a particular wavelength range (as opposed to the amount of a single wavelength that is absorbed).

Advantageously, the combination of the absorbent layer and the ultraviolet mirror in the multilayer article enables the use of a broadband UV light source to ultimately provide a relatively narrow band of UVC light (e.g., ranging from 195 nanometers to 240 nanometers). This is accomplished by 1) the ultraviolet mirror i) reflecting light having a wavelength ranging from 195 nm to as much as 240 nm and ii) transmitting light having a wavelength ranging from greater than the maximum wavelength of the reflecting range to 400 nm; and 2) the absorbent layer absorbing light having a wavelength ranging from 230 nm to 400 nm. As indicated above, typically the absorption, transmission, and/or reflection is less than 100% of the total incident light. **In** most preferred embodiments, greater than 90 percent, 91, 92, 93, 94, 95, 96, 97, or 98 or greater, of incident light is absorbed, transmitted, and/or reflected. Wavelengths of light below 230 nm have not been found to be carcinogenic to human skin, thus the reflection of 195 nm to 230 nm by the ultraviolet mirror can assist in disinfection with less risk to humans in the vicinity. Wavelengths of light between 240 nm and 230 nm may also be acceptable in some applications. **In** some embodiments, the multilayer article has a UV reflectivity greater than 90% (in some embodiments, greater than 99%), specifically of at least a wavelength of 222 nm.

The absorbent layer preferably resists ultraviolet light-induced damage/degradation over time by absorbing ultraviolet light that may pass through the ultraviolet mirror. Ultraviolet light, in particular the ultraviolet radiation having wavelengths in a range from 280 nm to 400 nm, can induce degradation of plastics, which in turn results in color change and deterioration of optical and mechanical properties. Inhibition of photo-oxidative degradation is important, for instance, for outdoor applications wherein long-term durability is mandatory. The absorption of ultraviolet light by polyethylene terephthalates, for example, starts at around 360 nm, increases markedly below 320 nm, and is very pronounced at below 300 nm. Polyethylene naphthalates strongly absorb ultraviolet light in the 310 nm to 370 nm range, with an absorption tail extending to about 410 nm, and with absorption maxima occurring at 352 nm and 337 nm. Chain cleavage occurs in the presence of oxygen, and the predominant photooxidation products are carbon monoxide, carbon dioxide, and carboxylic acids. Besides the direct photolysis of the ester groups, consideration has to be given to oxidation reactions, which likewise form carbon dioxide via peroxide radicals.

In general, the absorbent layer may include any polymeric composition (i.e., polymer plus additives) that is capable of withstanding ultraviolet light radiation for an extended period of time, while absorbing (including scattering) ultraviolet radiation. **In** some embodiments, the absorbent layer comprises a silicone thermoplastic, a fluoropolymer, copolymers thereof, or blends thereof. **In** some embodiments, the absorbent layer comprises a fluoropolymer (co)polymer comprising polymerized units derived from one or more monomers selected from tetrafluoroethylene, hexafluoropropylene, vinylidene fluoride, a perfluoroalkoxy alkylene, or a combination thereof. **In** this context, the term "polymer" will be understood to include homopolymers and copolymers, as well as polymers or copolymers that may be formed in a miscible blend, for example, by coextrusion or by reaction, including transesterification. The terms "polymer" and "copolymer" also include both random and block copolymers. These polymers, suitable for the absorbent layer, tend to exhibit less degradation from exposure to ultraviolet radiation (e.g., wavelengths between 195 nm and 400 nm) than other polymers formed of different monomers.

In some embodiments, the absorbent layer comprises one or more of an ultraviolet radiation absorber, an ultraviolet radiation scatterer, a hindered amine light stabilizer, an anti-oxidant, a pigment, or a combination thereof. Suitable ultraviolet radiation absorbers include carbon black, titanium dioxide, zinc oxide, cesium dioxide, zirconium dioxide, or combinations thereof. These particular ultraviolet radiation absorbers tend to be stable to ultraviolet radiation in addition to absorbing the radiation. Suitable ultraviolet radiation absorbers further include a benzotriazole compound, a benzophenone compound, a triazine compound (e.g., including any combination thereof).

Some suitable ultraviolet radiation absorbers are red shifted UV absorbers (RUVA) which absorb at least 70% (in some embodiments, at least 80%, or even greater than 90%) of the UV light in the wavelength region from 180 nm to 400 nm. Typically, it is desirable if the RUVA is highly soluble in polymers of the absorbent layer, highly absorptive, photo-permanent and thermally stable in the temperature range from 200°C to 300°C for extrusion process to form the protec.

RUVAs typically have enhanced spectral coverage in the long-wave UV region, enabling it to block the high wavelength UV light that can cause yellowing in polyesters. Typical UV protective layers have thicknesses in a range from 13 micrometers to 380 micrometers (0.5 mil to 15 mils) with a RUVA loading level of 2-10 wt.%. One of the most effective RUVA is a benzotriazole compound, 5-trifluoromethyl-2-(2-hydroxy-3-alpha-cumyl-5-tert-octylphenyl)-2H-benzotriazole (available under the trade designation "CGL-0139" from BASF, Florham Park, NJ). Other exemplary benzotriazoles include 2-(2-hydroxy-3,5-di-alpha-cumylphehyl)-2H-benzotriazole, 5-chloro-2-(2-hydroxy-3-tert-butyl-5-methylphenyl)-2H-benzotiazole, 5-chloro-2-(2-hydroxy-3,5-di-tert-butylphenyl)-2H-benzotriazole, 2-(2-hydroxy-3,5-di-tert-amylphenyl)-2H-benzotriazole, 2-(2-hydroxy-3-alpha-cumyl-5-tert-octylphenyl)-2H-benzotriazole, 2-(3-tert-butyl-2-hydroxy-5-methylphenyl)-5-chloro-2H-benzotriazole. Further exemplary RUVAs includes 2(-4,6-diphenyl-1-3,5-triazin-2-yl)-5-hexyloxy-phenol. Other exemplary UV absorbers include those available from BASF under the trade designations "TINUVIN 1577," "TINUVIN 900," "TINUVIN 1600," and "TINUVIN 777." Other exemplary UV absorbers are available, for example, in a polyester master batch under the trade designation "TA07-07 MB" from Sukano Polymers Corporation, Dunkin, SC. An exemplary UV absorber for polymethylmethacrylate is a masterbatch available, for example, under the trade designation "TA11-10 MBO1" from Sukano Polymers Corporation. An exemplary UV absorber for polycarbonate is a masterbatch from Sukano Polymers Corporation, under the trade designations "TA28-09 MB01." In addition, the UV absorbers can be used in combination with hindered amine light stabilizers (HALS) and anti-oxidants. Exemplary HALS include those available from BASF, under the trade designation "CHIMASSORB 944" and "TINUVIN 123." Exemplary anti-oxidants include those obtained under the trade designations "IRGANOX 1010" and "ULTRANOX 626", also available from BASF.

In select embodiments, the absorbent layer further absorbs at least 30 percent, 40, 50, 60, 70, 80, or at least 90 percent of incident visible light having a wavelength between at least 400 nm and 700 nm. Typically, there is no need for the multilayer article to be transparent to visible light, thus it can be preferred for the absorbent layer to absorb 70 percent or greater of incident visible light having a wavelength between at least 400 nm and 700 nm to minimize reflection of visible light back out of the multilayer article.

The ultraviolet mirror comprises multiple low/high index pairs of film layers, wherein each low/high index pair of layers has a combined optical thickness of 1/2 the center wavelength of the band it is designed to reflect. Stacks of such films are commonly referred to as quarterwave stacks. In some embodiments, different low/high index pairs of layers may have different combined optical thicknesses, such as where a broadband reflective optical film is desired. Materials employed in the ultraviolet mirrors are preferably resistant to ultraviolet radiation. Many fluoropolymers and certain inorganic materials are resistant to ultraviolet radiation.

In some embodiments of the ultraviolet mirrors described herein, the at least first optical layer comprises inorganic material (e.g., at least one of zirconium oxynitride, hafnia, alumina, magnesium oxide, yttrium oxide, lanthanum fluoride, or neodymium fluoride), and wherein the second optical layer comprises inorganic material (e.g., at least one of silica, aluminum fluoride, magnesium fluoride, calcium fluoride, silica alumina oxide, or alumina doped silica). Exemplary materials are available, for example, from Materion Corporation, Mayfield Heights, OH, and Umicore Corporation, Brussels, Belgium.

In some embodiments of the ultraviolet mirrors described herein, the at least first optical layer comprises a polymeric material (e.g., at least one of polyvinylidene fluoride (PVDF), ethylene tetrafluoroethylene (ETFE)), and the second optical layer comprises polymeric material (e.g., at least one of a copolymer (THV,) or a polyethylene copolymer comprising subunits derived from tetrafluoroethylene (TFE), hexafluoropropylene (HFP), and vinylidene fluoride (VDF), a copolymer (FEP) comprising subunits derived from tetrafluoro-ethylene (TFE) and hexafluoropropylene (HFP), or perfluoroalkoxy alkane (PFA)).

Second optical layers can comprise fluorinated copolymers materials such as at least one of fluorinated ethylene propylene copolymer (FEP); copolymers of tetrafluorethylene, hexafluoropropylene, and vinylidene fluoride (THV); copolymers of tetrafluoroethylene, hexafluoropropylene, or ethylene. Particularly useful are melt processible copolymers of tetrafluoroethylene and at least two, or even at least three, additional different comonomers.

In some embodiments, the first optical layer is a fluoropolymer and the second optical layer is a fluoropolymer. Examples of the materials that are desirable for such embodiments include ETFE/THV, PMMA/THV, PVDF/FEP, ETFE/FEP, PVDF/PFA, and ETFE/PFA. In select embodiments, the at least first optical layer comprises at least one of polyvinylidene fluoride or ethylene tetrafluoroethylene (ETFE) and the second optical layer comprises a copolymer of tetrafluoroethylene, hexafluoropropylene, and vinylidene fluoride (THV).

Exemplary melt processible copolymers of tetrafluoroethylene and other monomers discussed above include those available as copolymers of tetrafluoroethylene, hexafluoropropylene, and vinylidene fluoride under the trade designations "DYNEON THV 220," "DYNEON THV 230," "DYNEON THV 2030," "DYNEON THV 500," "DYNEON THV 610," and "DYNEON THV 815" from Dyneon LLC, Oakdale, MN; "NEOFLON EFEP" from Daikin Industries, Ltd., Osaka, Japan; "AFLAS" from Asahi Glass Co., Ltd., Tokyo, Japan; and copolymers of ethylene and tetrafluoroethylene available under the trade designations "DYNEON ET 6210A" and "DYNEON ET 6235" from Dyneon LLC; "TEFZEL ETFE" from E.I. duPont de Nemours and Co., Wilmington, DE; and "FLUON ETFE" by Asahi Glass Co., Ltd.

Ultraviolet mirrors described herein can be made using general processing techniques, such as by coextrusion of alternating polymer layers having different refractive indices, for example, as described in U.S Pat. Nos. 5,882,774 (Jonza et al.); 6,045,894 (Jonza et al.); 6,368,699 (Gilbert et al.); 6,531,230 (Weber et al.); 6,667,095 (Wheatley et al.); 6,783,349 (Neavin et al.); 7,271,951 B2 (Weber et al); 7,632,568 (Padiyath et al.); 7,652,736 (Padiyath et al.); and 7,952,805 (McGurran et al.); and PCT Publications WO 95/17303 (Ouderkirk et al.) and WO 99/39224 (Ouderkirk et al.).

Desirable techniques for providing an ultraviolet mirror with a controlled spectrum include the use of an axial rod heater control of the layer thickness values of coextruded polymer layers as described, for example, in U.S. Pat. No. 6,783,349 (Neavin et al.); timely layer thickness profile feedback during production from a layer thickness measurement tool such as an atomic force microscope (AFM), a transmission electron microscope, or a scanning electron microscope; optical modeling to generate the desired layer thickness profile; and repeating axial rod adjustments based on the difference between the measured layer profile and the desired layer profile.

The basic process for layer thickness profile control involves adjustment of axial rod zone power settings based on the difference of the target layer thickness profile and the measured layer profile. The axial rod power increase needed to adjust the layer thickness values in a given feedblock zone may first be calibrated in terms of watts of heat input per nanometer of resulting thickness change of the layers generated in that heater zone. For example, fine control of the spectrum is possible using 24 axial rod zones for 275 layers. Once calibrated, the necessary power adjustments can be calculated once given a target profile and a measured profile. The procedure is repeated until the two profiles converge.

The layer thickness profile (layer thickness values) of ultraviolet mirrors described herein reflecting at least 50 percent of incident UV light over a specified wavelength range can be adjusted to be approximately a linear profile with the first (thinnest) optical layers adjusted to have about a 1/4 wave optical thickness (index times physical thickness) for 190 nm light and progressing to the thickest layers which would be adjusted to be about 1/4 wave thick optical thickness for 240 nm light or 230 nm light.

Dielectric mirrors, with optical thin film stack designs comprised of alternating thin layers of inorganic dielectric materials with refractive index contrast, are particularly suited for this. In recent decades they are used for applications in UV, Visible, NIR and IR spectral regions. Depending upon the spectral region of interest, there are specific materials suitable for that region. Also, for coating these materials, one of two forms of physical vapor deposition (PVD) are used: evaporation or sputtering. Evaporated coatings rely upon heating the coating material (evaporant) to a temperature at which it evaporates. This is followed by condensation of the vapor upon a substrate. For evaporated dielectric mirror coatings, the electron-beam deposition process is most commonly used. Sputtered coatings use energetic gas ions to bombard a material ("target") surface, ejecting atoms which then condense on the nearby substrate. Depending upon which coating method is used, and the settings used for that method, thin film coating rate and structure-property relationships will be strongly influenced. Ideally, coating rates should be high enough to allow acceptable process throughput and film performance, characterized as dense, low stress, void free, non-optically absorbing coated layers.

Exemplary embodiments can be designed to have peak reflectance at 222 nm, by both PVD methods. For example, coating discrete substrates by electron-beam deposition method, using $HfO_2$ as the high refractive index material and $SiO_2$ as the low refractive index material. Mirror design has alternating layers of "quarter wave optical thickness" (qwot) of each material, that are coated, layer by layer until, for example, after 11 layers the reflectance at 215 nm is > 95%. The bandwidth of this reflection peak is around 50 nm. Quarter wave optical thickness is the design wavelength, here 215 nm, divided by 4, or 53.75 nm. Physical thickness of the high refractive index layers ($HfO_2$) is the quotient of qwot and refractive index of $HfO_2$ at 215 nm (2.35), or 23.2 nm. Physical thickness of the low refractive index layers ($MgF_2$), with 215 nm refractive index at 1.42, is 37.85 nm. Coating a thin film stack, then, which is comprised of alternating layers of $HfO_2$ and $SiO_2$ and designed to have peak reflectance at 215 nm begins by coating layer 1 $HfO_2$ at 23.2 nm. In electron beam deposition a four-hearth evaporation source is used. Each hearth is cone-shaped and 17 $cm^3$ volume of $HfO_2$ chunks fill it. The magnetically deflected high voltage electron beam is raster scanned over the material surface as filament current of the beam is steadily, in a pre-programmed fashion, increased. Upon completion of the pre-programmed step the $HFO_2$ surface is heated to evaporation temperature, about 2500°C, and a source shutter opens, the $HfO_2$ vapor flux emerging from the source in a cosine-shaped distribution and condensing upon the substrate material above the source. For enhancement of coating uniformity, the substrate holders rotate during deposition. Upon reaching the prescribed coating thickness (23.2 nm) the filament current shuts off; the shutter closes and the $HfO_2$ material cools. For layer 2 the evaporation source is then rotated to a hearth containing chunks of $MgF_2$ and a similar pre-programmed heating process begins. Here, the $MgF_2$ surface temperature is about 950°C when the source shutter opens and, upon reaching the prescribed coating thickness (37.85 nm), the filament current shuts off; the shutter closes and the $HfO_2$ material cools. This step-wise process is continued, layer by layer, until the total number of design layers is reached. With this optical design, as total layers are increased, from 3 to 11, the resulting peak reflectance increases accordingly, from 40% at 3 layers to > 95% at 11 layers.

Optionally, ultraviolet mirrors can be prepared in continuous roll to roll (R2R) fashion, using ZrON as the high refractive index material and $SiO_2$ as the low refractive index material. The optical design is the same type of thin film stack, alternating qwot layers of the two materials. For ZrON, with refractive index at 215 nm of 3.1, the physical thickness target was 17.3 nm. For $SiO_2$, here sputtered from an aluminum-doped silicon sputter target, with refractive index 1.61, the target thickness was 33.3 nm. Layer one ZrON is DC sputtered from a pure zirconium sputter target in a gas mixture of argon, oxygen and nitrogen. Whereas argon is the primary sputtering gas, oxygen and nitrogen levels are set to achieve transparency, low absorptance and high refractive index. The film roll transport initially starts at a pre-determined speed, and the sputter source power is ramped to full operating power, followed by introduction of the reactive gases and then achieving steady state condition. Depending upon the length of film to coat, the process continues until total footage is achieved. Here, as the sputter source is orthogonal to and wider than the film which is being coated, the uniformity of coating thickness is quite high. Upon reaching the desired length of coated film the reactive gases are set to zero and the target is sputtered to a pure Zr surface state. The film direction is next reversed and silicon (aluminum doped) rotary pair of sputter targets has AC frequency (40 kHz) power applied in an argon sputtering atmosphere. Upon reaching steady state, oxygen reactive gas is introduced to provide transparency and low refractive index. At the pre-determined process setting and line speed the second layer is coated over the length which was coated for layer one. Again, as these sputter sources are also orthogonal to and wider than the film being coated, the uniformity of coating thickness is quite high. After reaching the desired length of coated film the reactive oxygen is removed and the target is sputtered in argon to a pure silicon (aluminum doped) surface state. Layers three to five or seven or nine or eleven or thirteen, depending upon peak reflectance target, are coated in this sequence. Upon completion, the film roll is removed for post-processing.

For manufacturing of these inorganic coatings, the electron beam process is best suited for coating discrete parts. Though some chambers have demonstrated R2R film coating, the layer by layer coating sequence would still be necessary. For R2R sputtering of film, it is advantageous to use a sputtering system with multiple sources located around one, or perhaps two, coating drums. Here, for a thirteen layers optical stack design, a two, or even single, machine pass process, with alternating high and low refractive index layers coated sequentially, would be feasible. How many machine passes needed would be contingent upon machine design, cost, practicality of thirteen consecutive sources, and so forth. Additionally, coating rates would need to be matched to a single film line speed.

Preferably, the ultraviolet mirror reflects at an incident light angle of at least one of 0°, 15°, 30°, 45°, 60°, or 75°, at least 80 percent, 85, 90, 91, 92, 93, 94, 95, 96, 97, or at least 98 percent of incident ultraviolet light in a wavelength range from 200 nanometers to 230 nanometers. The selection of the material combinations used in creating the ultraviolet mirror depends, for example, upon the desired bandwidth that will be reflected. Higher refractive index differences between the first optical layer polymer and the second optical layer polymer create more optical power thus enabling more reflective bandwidth per pair of layers. The number of optical layers is selected to achieve the desired optical properties using the minimum number of layers for reasons of film thickness, flexibility and economy. **In** the case of reflective films such as mirrors, the number of layers is preferably less than about 2,000, more preferably less than about 1,000, and even more preferably less than about 750. **In** some embodiments, the number of layers is at least 100, 125, 150, 175, or at least 200. The refractive index of zirconia, however, is so high that a much lower number of optical layers is needed when zirconia or zirconia oxynitride is employed, such as 50 optical layers or less, 40, 30, 20, or 15 optical layers or less; and 3 optical layers or more, 5, 7, or 10 optical layers or more, may be needed.

In some embodiments, the ultraviolet mirror has a reflection spectrum at an incident light angle of 0° (e.g., normal incidence) that shifts to shorter wavelengths at oblique angles (e.g., 15°, 30°, 45°, 60°, or 75°). One can thus prepare an ultraviolet mirror having a normal incidence spectrum such that at an intended angle of incidence, the ultraviolet mirror reflects ultraviolet light in a range of 195 nm to 240 nm. Optionally, an intervening optical element (e.g., prism, louver, or the like) is placed between the ultraviolet mirror and a UVC light source to change or limit the angle of incidence of the light emitted by the UVC light source before it reaches an exterior surface of the ultraviolet mirror. Moreover, one can form a shape of an exterior surface of the ultraviolet mirror such that the angle of incidence is maintained for various locations of the ultraviolet mirror.

In some embodiments, the ultraviolet mirror absorbs at an incident light angle of at least one of 0°, 15°, 30°, 45°, 60°, or 75°, at least 30 percent, 40, 50, 60, 70, 80, 90 percent, at least 95 percent, or at least 98 percent of incident visible light having a wavelength between at least 400 nm and 700 nm. Optionally, a pigment or a dye can be included in the ultraviolet mirror to absorb one or more wavelengths from 400 nm to 700 nm. Suitable pigments may include for instance, metal oxides such as, for example, antimony tin oxide, indium tin oxide, cesium oxides, iron oxides, and cuprous oxides. As mentioned above with respect to the absorbent layer, there is typically no need for the multilayer article to be transparent to visible light, thus it can be preferred for the ultraviolet mirror to absorb 30 percent or greater of incident visible light having a wavelength between at least 400 nm and 700 nm to minimize reflection of visible light back out of the multilayer article.

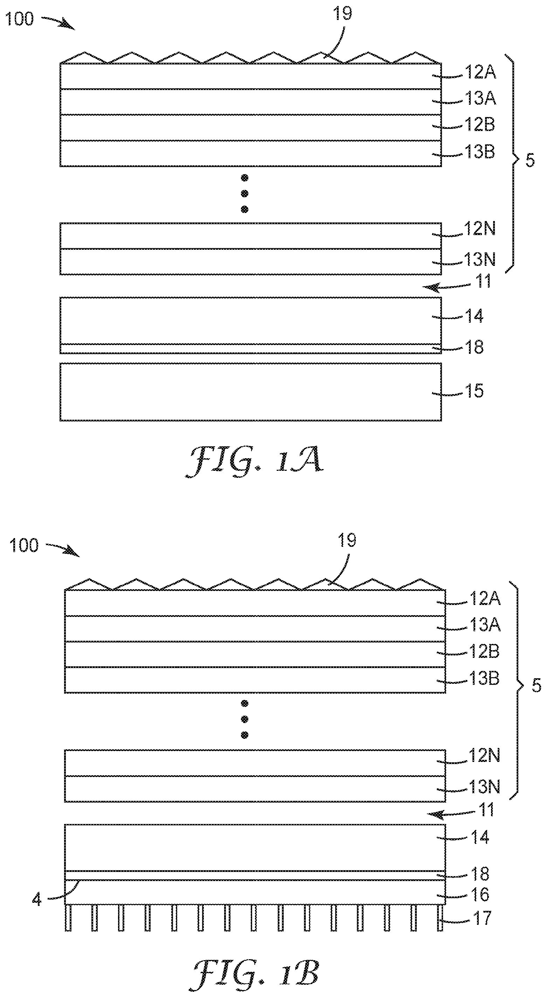

Referring to FIGS. 1A and 1B, schematic cross-sectional views are provided of two exemplary multilayer articles 100, each including an ultraviolet mirror 5 comprising first optical layers 12A, 12B, 12N, second optical layers 13A, 13B, 13N and an absorbent layer 14 adjacent to the ultraviolet mirror.

In some embodiments, a major surface of the absorbent layer 14 is in direct contact with a major surface of the ultraviolet mirror 5. In other embodiments, the multilayer article 100 comprises an air gap 11 disposed between the absorbent layer 14 and the ultraviolet mirror 5, as shown in the figures. For instance, an air gap can be achieved by taping the edges of the absorbent layer to the ultraviolent mirror. Attaching the two layers using adhesive tape also enables using an ultraviolet mirror having a shape that is different than the shape of the absorbent layer.

Referring to FIG. 1A, the multilayer article 100 optionally further comprises an adhesive layer 15 adjacent to the absorbent layer 14, wherein the absorbent layer 14 is disposed between the ultraviolet mirror 5 and the adhesive layer 15. An adhesive could be useful for attaching the multilayer article to a substrate (e.g., a wall, a ceiling, a device housing, etc.). Such optional adhesive layers may comprise any adhesive (e.g., thermosetting adhesive, hot melt adhesive, and/or pressure-sensitive adhesive). If present, an optional adhesive layer preferably comprises a pressure-sensitive adhesive. In some embodiments, the adhesive may be resistant to ultraviolet radiation damage. Exemplary adhesives which are typically resistant to ultraviolet radiation damage include silicone adhesives and acrylic adhesives containing UV-stabilizing/blocking additive(s), for example, as discussed hereinabove. The optional adhesive layer may comprise thermally-conductive particles to aid in heat transfer. Exemplary thermally-conductive particles include aluminum oxide particles, alumina nanoparticles, aluminum trihydrate, aluminum coated glass beads, metal silicides, graphite, graphene, carbon nanotubes, hexagonal boron nitride particles and agglomerates (e.g., available as 3M BORON DINITRIDE from 3M Company), graphene particles, graphene oxide particles, metal particles, and combinations thereof. Further, optional releasable liners used with an optional adhesive layer may comprise, for example, a polyolefin film, a fluoropolymer film, a coated PET film, or a siliconized film or paper.

Referring to FIG. 1B, the multilayer article optionally further comprises one or more of a heat transfer layer 16, a plurality of heat transfer fins 17, or a plurality of heat transfer pins 17, adjacent to a major surface 4 of the absorbing layer 14 and opposite the ultraviolet mirror 5. Suitable materials of which the heat transfer layer, fins, and/or pins may be composed includes metals such as aluminum, silver, gold, copper, nickel, iron, steel, or titanium. The heat transfer layer, plurality of heat transfer fins, or plurality of heat transfer pins, may comprise a polymer filled with thermally-conductive particles including; aluminum oxide particles, alumina nanoparticles, aluminum trihydrate, aluminum coated glass beads, metal silicides, graphite, graphene, carbon nanotubes, hexagonal boron nitride particles and agglomerates (e.g., available as 3M BORON DINITRIDE from 3M Company), graphene particles, graphene oxide particles, metal particles, and combinations thereof.

Referring again to each of FIGS. 1A-1B, additional optional features are provided. For instance, in some embodiments, an (e.g., outer) major surface of the ultraviolet mirror 5 may comprise a plurality of nonplanar features 19 protruding from the major surface. Any shape of nonplanar features may be suitable, (e.g., prisms, ridges, linear and/or curved polygons). In the embodiment shown, the nonplanar features 19 have a shape of a triangular prism. Such nonplanar features may be micro-structured and/or nano-structured over some or all of its surface; for example, as described in PCT International Application Publication No. WO 2019/130198 (Hebrink et al.). In some embodiments, the nano-structure may be superimposed on the microstructure on the surface of the ultraviolet mirror. The micro-structures may be arranged as a series of alternating micro-peaks and micro-spaces. The size and shape of the micro-spaces between micro-peaks may mitigate the adhesion of dirt particles to the micro-peaks. The nano-structures may be arranged as at least one series of nano-peaks disposed on at least the micro-spaces. The micro-peaks may be more durable to environmental effects than the nano-peaks. Because the micro-peaks are spaced only by a micro-space, and the micro-spaces are significantly taller than the nano-peaks, the micro-peaks may serve to protect the nano-peaks on the surface of the micro-spaces from abrasion. Moreover, the nonplanar features may act as light diffusive structures by scattering UVC light reflected from the ultraviolet mirror.

In some embodiments, the ultraviolet mirror may comprise structures specifically for providing light diffusion, for instance when the reflected light is being directed into an area (e.g., a room) in which people may be present. Such light diffusive structures may be provided by including inorganic particles. For example, each structure may correspond to one inorganic particle. The inorganic particles may be dispersed in or disposed on at least one layer of the ultraviolet mirror. The inorganic particles may comprise titania, silica, zirconia, or zinc oxide. The inorganic particles may be in the form of beads or microbeads. The inorganic particles may be formed of a ceramic material, glass, or various combinations of thereof. In some embodiments, the inorganic particles have an effective $D_{90}$ particle size of at least 1 (in some embodiments, at least 3, 5, 6, 7, 8, 9, 10, or even at least 20) micrometers. In some embodiments, the inorganic particles have an effective $D_{90}$ particle size of at most 40 (in some embodiments, at most 25, 20, 15, 14, 13, 12, 11, 10, 9, or even at most 8) micrometers. Surface structures may also include cross-linked polymer beads such as those under the tradename "CHEMISNOW" available from Soken Chemical & Engineering Company, Tokyo, Japan. As defined in NIST "Particle Size Characterization", ASTM B15-96 describes $D_{90}$ as the intercept where 90% of the samples mass has particles with a diameter less than the value. For example, a $D_{90}$ of 10 micrometers specifies that 90% of the samples mass includes particles with diameters less than 10 micrometers.

In some embodiments, the absorbent layer 14 comprises a continuous metal coating or layer 18. Suitable coating or layer thicknesses include 50 nm or greater, 55 nm, 60 nm, 65 nm, 70 nm, or 75 nm or greater; and 100 nm or less, 95 nm, 90 nm, 85 nm, or 80 nm or less. In some embodiments, the continuous metal coating or layer is the entire absorbent layer 14, whereas in the embodiment shown in FIGS. 1A-1B, the continuous metal coating or layer is used in combination with a polymeric absorbent material. In some embodiments, the absorbent layer comprises metal particles disposed in a polymer matrix (not shown). The size of the metal particles is not particularly limited, and the average particle size of the metal particles can range from 10 nm to 10000 nm (10 micrometers). Suitable metals for use as a coating, a layer, or a plurality of particles, include one or more of silver, gold, copper, nickel, or titanium. Use of metal in certain embodiments can increase the absorption of incident light having a wavelength between at least 400 nm and 700 nm (e.g., visible light). Further, the metal can either scatter or absorb harmful UV radiation thereby reducing damage to thermoplastics. U.S Pat. No. 5,504,134 (Palmer et al.), for instance, describes attenuation of polymer substrate degradation due to ultraviolet radiation through the use of metal oxide particles in a size range of about 0.001 to about 0.2 micrometers (in some embodiments, about 0.01 micrometers to about 0.15 micrometers) in diameter. U.S. Pat. No. 5,876,688 (Laundon), describes a method for producing micronized zinc oxide that are small enough to be transparent when incorporated as UV blocking and/or scattering agents in paints, coatings, finishes, plastic articles, cosmetics and the like which are well suited for use in the present invention. These fine particles such as zinc oxide and titanium oxide with particle sizes ranging from 10 nm to 100 nm that can attenuate UV radiation are available, for example, from Kobo Products, Inc., South Plainfield, NJ. Flame retardants may also be incorporated as an additive in an absorbent layer.

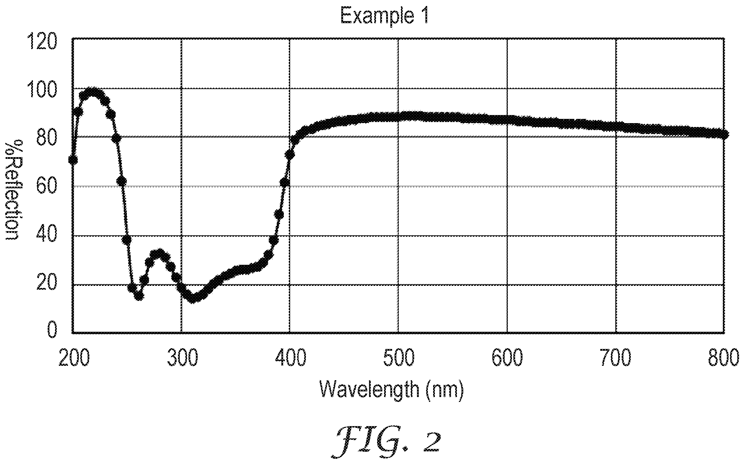

Referring to FIG. 2, a graph is provided showing percent reflection as a function of wavelength of the multilayer article of Preparative Example 1, which is described in detail in the Examples below. FIG. 2 shows high percent reflection, namely an average percent reflection of 89.9% over the wavelength range of 200 nm to 240 nm.

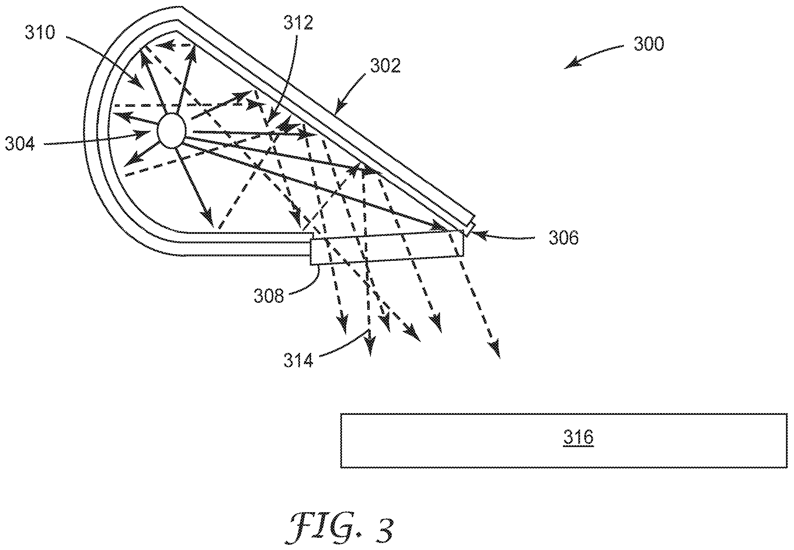

Referring to FIG. 3, a schematic cross-sectional view is provided of an exemplary device 300, according to the present disclosure. The device 300 comprises a housing 302 that is substantially impermeable to ultraviolet radiation having a wavelength of from 280 nm to 400 nm, at least one window 308 defined in the housing, and a broadband UVC light source 304 positioned within the housing. The broadband UVC light source 304 is capable of emitting ultraviolet radiation 310 at one or more wavelength from 100 nm to 400 nm. The device 300 further includes a multilayer article 306 positioned within the housing 302 so as to reflect 312 ultraviolet radiation 310 emitted by the broadband UVC light source 304. The multilayer article 306 (see FIGS. 1A-1B) is as described in detail above.

The window 308 may be formed of any material that allows passage of the ultraviolet radiation 314 out of the device 300. Suitable materials include for instance, pure silica, quartz, borosilicate, and a fluoropolymer. In some cases, a suitable window could provide functionality, such as by using a collimator or a diffuser (e.g., each as described in more detail below) as the window material. Such a window could be included in a device of any embodiment of the present disclosure. Alternatively, the window 308 can be an open aperture defined by the housing 302. The ultraviolet radiation 314 travels out of the device 300, transmitting ultraviolet radiation 314 having a wavelength range of 195 nanometers or 200 nm, to 230 nanometers, 235 nm, or 240 nm. The ultraviolet radiation 314 emitted from the device 300 may be directed at a material 316 to be disinfected to a desired level of disinfection.

Typically, the housing 302 is configured to block direct passage of light 310 from the broadband UVC light source 304 through the window 308, with the exception of light that travels a distance of at least 3 centimeters (cm), 3.25 cm, 3.5 cm, 3.75 cm, or at least 4 cm from the broadband UVC light source 304 to the window 308. Such a distance is sufficiently long to decrease the harmfulness of any stray light 310 that does not have the high wavelengths of light get absorbed by the multilayer article 306. In certain cases, the housing 302 is configured to block direct passage of light 310 from the broadband UVC light source 304 through the window 308 entirely.

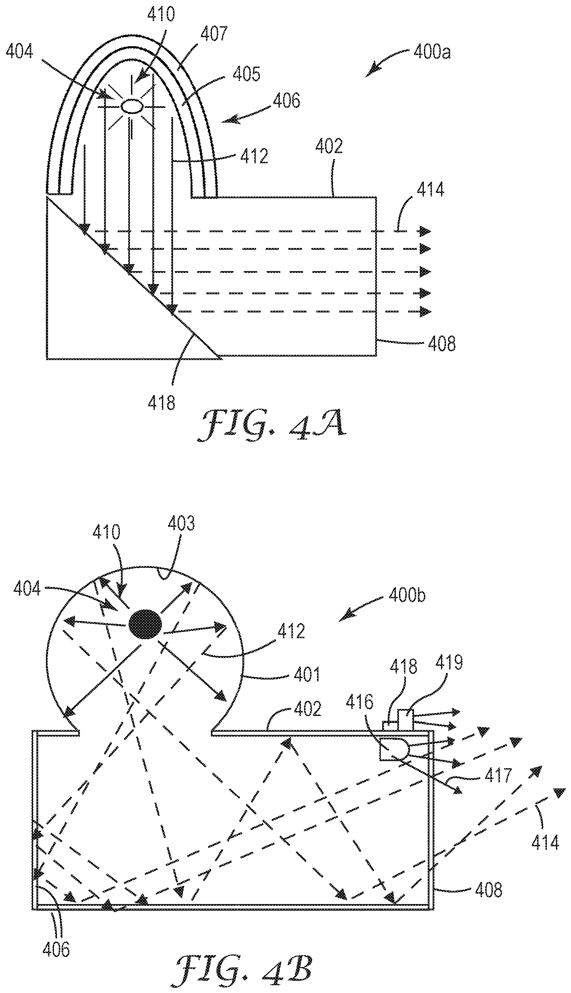

Referring to FIG. 4A, a schematic cross-sectional view is provided of another exemplary device 400a, according to the present disclosure. The device 400a comprises a housing 402 that is substantially impermeable to ultraviolet radiation having a wavelength of from 280 nm to 400 nm, at least one window 408 defined in the housing, and a broadband UVC light source 404 positioned within the housing 402. The broadband UVC light source 404 is capable of emitting ultraviolet radiation 410 at one or more wavelength from 100 nm to 400 nm. In this embodiment, the housing 402 is configured to completely block direct passage of light 410 from the broadband UVC light source 404 through the window 408.

The device 400a further includes a multilayer article 406 positioned within the housing 402 so as to reflect 412 ultraviolet radiation 410 emitted by the broadband UVC light source 404. The multilayer article 406 (see FIGS. 1A-1B) is as described in detail above, and here is depicts both the ultraviolet mirror 405 and the absorbent layer 407, where the broadband UVC light source 404 is configured to direct light 410 at the ultraviolet mirror 405 of the multilayer article 406.

The device 400a further comprises a reflector 418 configured to direct the reflected ultraviolet radiation 412 towards the window 408. The ultraviolet radiation 414 travels out of the device 400a through the window 408, transmitting ultraviolet radiation 414 having a wavelength range of 195 nanometers or 200 nm, to 230 nanometers, 235 nm, or 240 nm. The reflector 418 may include any material that will effectively reflect the reflected ultraviolet radiation 412 wavelengths (e.g., between 195 nm and 240 nm), for instance a UVC reflective material such as a multilayer article (as described in detail above) or a mirror (e.g., polished aluminum). The ultraviolet radiation 414 emitted from the device 400a may be directed at a material (not shown) to be disinfected to a desired level of disinfection.

Referring to FIG. 4B, a schematic cross-sectional view is provided of an additional exemplary device 400b, according to the present disclosure. The device 400b comprises a housing 402 that is substantially impermeable to ultraviolet radiation having a wavelength of from 280 nm to 400 nm, at least one window 408 defined in the housing, and a broadband UVC light source 404 positioned in an enclosure 401 adjacent to the housing 402. In this embodiment, the enclosure 401 is attached directly to the housing 402 (although this may not be necessary if the enclosure and the housing are configured such that none of the ultraviolet radiation emitted from the broadband UVC source exits the device before first interacting with a multilayer article as described in detail above).

The broadband UVC light source 404 is capable of emitting ultraviolet radiation 410 at one or more wavelength from 100 nm to 400 nm. The enclosure 401 comprises a major surface 403 located on the interior surface of the enclosure 401. The major surface 403 comprises a material that reflects at least a portion of the emitted ultraviolet radiation 410, for instance a mirror, to assist in directing ultraviolet radiation 410 into the housing 402 as reflected ultraviolet radiation 412. Optionally, the major surface 403 comprises a multilayer article such as the multilayer article described in detail above (e.g., as shown in FIGS. 1A-1B). Further, in this embodiment, each of the enclosure 401 and the housing 402 is configured to completely block direct passage of light 410 from the broadband UVC light source 404 through the window 408 of the housing 402.

The device 400b further includes a multilayer article 406 positioned within the housing 402 so as to reflect the ultraviolet radiation 410 and to further reflect the reflected ultraviolet radiation 412 received from the enclosure 401. The multilayer article 406 (see FIGS. 1A-1B) is as described in detail above, in which ultraviolet radiation 410 and reflected ultraviolet radiation 412 are directed at the ultraviolet mirror (as opposed to the absorbent layer) of the multilayer article 406 (neither the ultraviolet mirror nor the absorbent layer are shown in FIG. 4B).

The ultraviolet radiation 414 travels out of the device 400b through the window 408, transmitting ultraviolet radiation 414 having a wavelength range of 195 nanometers or 200 nm, to 230 nanometers, 235 nm, or 240 nm. The ultraviolet radiation 414 emitted from the device 400b may be directed at a material (not shown) to be disinfected to a desired level of disinfection.

In any device according to the present disclosure, a visible light source may be provided that is configured to emit visible light when the UVC light source is emitting light. UVC light is not visible to the human eye, thus having visible light emitted has the safety benefit of informing a user that the UVC light is being emitted at that time. A visible light source 416, which emits visible light 417, is shown to be included in the device 400b of FIG. 4B. The visible light source 416 may be electrically powered (e.g., by the UVC light source 404) or may be optically powered (e.g., by the ultraviolet radiation 410 and reflected ultraviolet radiation 412). An exemplary visible light source 416 that is optically powered includes, for instance, a down converter, which absorbs ultraviolet radiation and emits visible radiation. Often, an activation switch 418 is present to allow a user to cause the visible light source 416 to emit light 417 or to halt emitting light 417 (e.g., turn on or turn off, respectively) the visible light source 416 when the activation switch 418 is engaged. For instance, the activation switch 418 can be positioned on the device 400b in a location that is accessible to a user (e.g., on an exterior surface of the housing 402). In some cases, the activation switch 418 is (e.g., electrically) connected to a motion detector 419. The motion detector 419 can be configured to turn on the visible light source 416 when motion is detected to provide an indication that the device 400b is in use, for instance when a person approaches the device 400b.

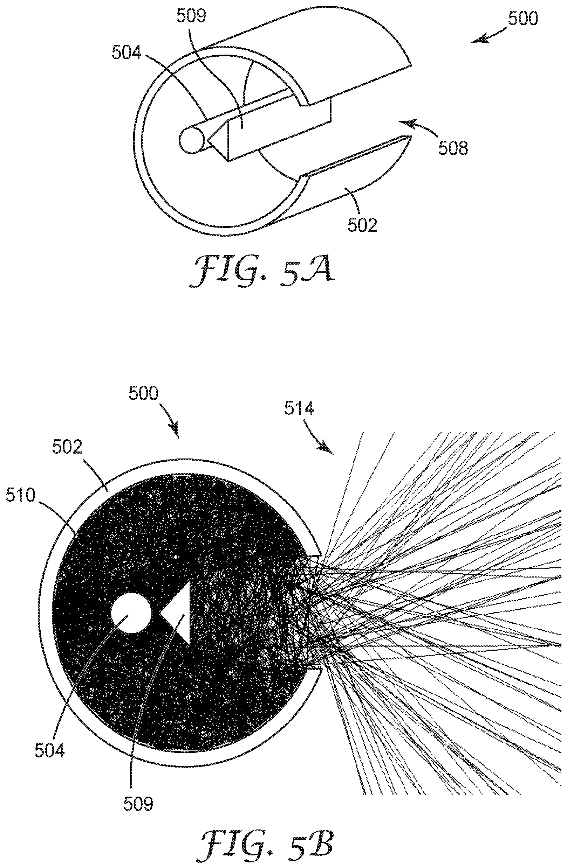

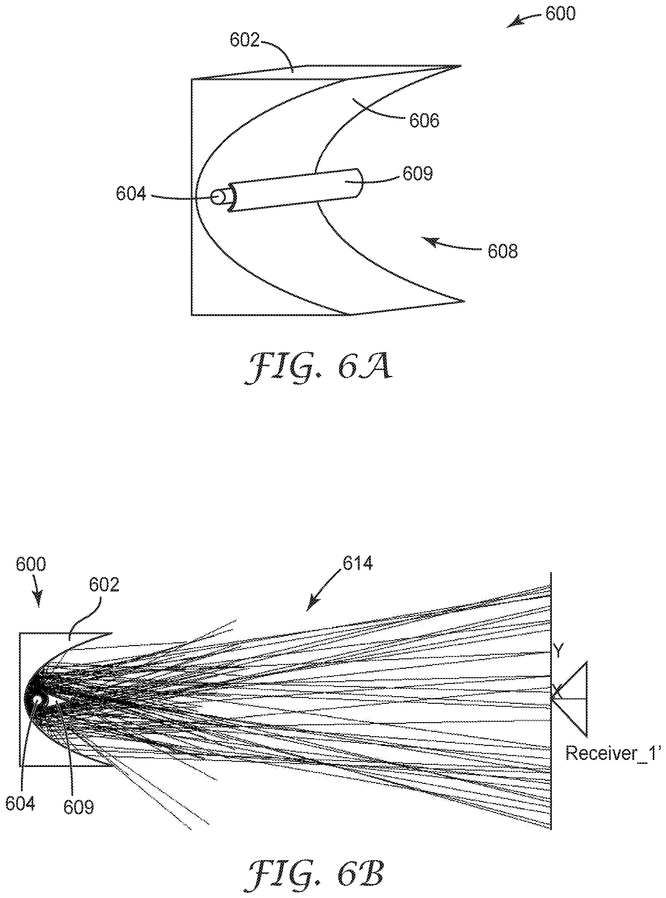

The shape of the housing for a device according to the present disclosure can vary; in some cases, a cross-section of at least a portion of the housing forms a semicircular shape, a circular shape, an elliptical shape, a parabolic shape or a rectangular shape, typically with a portion of the shape missing, which defines a window. For instance, a cross-section of a portion of the housing 302 of the device 300 of FIG. 3 forms a hemispherical shape and a cross-section of a portion of the housing 402 of each of the devices 400a and 400b forms a rectangular shape. Referring to FIGS. 5A-5B, a cross-section of a portion of a housing 502 of a device 500 forms a circular shape. Referring to FIGS. 6A-6B, a cross-section of a portion of a housing 602 of a device 600 forms a parabolic shape.

The material(s) of which the housing are composed are not particularly limited, and may include for instance metal, plastic, ceramic (including glass), concrete, or wood. In certain embodiments, the housing is formed of a heat-resistant or heat-transfer material that can withstand heat generated by absorption of certain wavelengths of light from the broadband UVC light source within the housing.

As mentioned above, "UVC" refers to wavelengths of light in a range between 100 nm and 280 nm. Broadband UVC light sources provide a band of wavelengths within this C wavelength range of 30 nm or greater, as opposed to providing a smaller band of wavelengths (e.g., as can be provided by a light emitting diode (LED) light source). Typically, in devices according to the present disclosure, the broadband UVC light source is configured to direct light at the ultraviolet mirror of the multilayer article. This allows the ultraviolet mirror to reflect back wavelengths of light in the desirable range (e.g., 195 nm to 240 nm) while transmitting to the absorbent layer and/or absorbing wavelengths of light greater than the maximum of the range (e.g., greater than 240 nm).

**In** any device embodiment, at least one angular control element may be disposed in the housing, e.g., located between the UVC light source and a window of the housing. A suitable material for an angular control element is a fluoropolymer. Typically, such an optional (at least one) angular control element comprises at least one of a collimator, a retroreflector, a diffuser, or a reflecting diverter, for instance a lens. **In** some embodiments, the angular control element comprises a solid structure, while in others it comprises a hollow structure. The angular control element may be in the form a film and is often linearly continuous. Preferably, the angular control element has rotational symmetry. **In** select embodiments, the angular control element comprises a concave shape. **In** some such embodiments, the broadband UVC light source is disposed between a first concave shape formed by the housing and a second concave shape formed by the angular control element, in which the first concave shape is curved in an opposing direction than the second concave shape.

**In** one embodiment of a device according to the present disclosure, the UVC collimator is a cuspate lens that redirects light laterally via total internal reflection. Such cuspate lenses generally have a depression in the center to form the cuspate, the shape of which ensures total internal reflection and lateral redirection for incident light. One suitable cuspate lens has a cuspate-torus combination and is as described in "The Black HoleTM: Cuspated waveguide-injectors and illuminators for LEDs", Parkyn et al., Proc. SPIE 3781, Nonimaging Optics: Maximum Efficiency Light Transfer V, (6 October 1999), in section 5 ("Lighthouse" Equatorial Outputs from Hemispheric Sources).

Light from a point source can be collimated (focused) using a parabolic (elliptical) reflective optical element, and one suitable collimator for the system comprises a parabolic collimator. The main requirements are that the source be located near the focal point of the optical element and that the source be relatively small compared with the size of the optical element. Light concentrators can be designed utilizing a surface of revolution generated from a section of an ellipse with the source at one focus and the target at the other focus of the ellipse. The source at one focus shines toward the closest vertex of the ellipse. The section of the ellipse used to generate the surface of revolution is the section defined by the latus rectum at the source and the closest vertex to the source. The latus rectum must be larger than the source so that the concentrator can collect most of the light from the source. If the source and target were points, all the light from the source would be collected at the target.

In most applications, the optical element must be designed for practical considerations such as the size of the light source and the allowed amount of space of the optical element. Given a source diameter $Ds$ (width in 1D) and a design volume consisting of a height Hv and diameter Dv (width in 1D), it is possible to derive an equation for the shape of a near-optimum parabolic reflector:

$$y = a * (x + b)^2 + offset$$

where $a = Hv / ((Dv/2)^2 - (Ds/2)^2)$, $b = -Dv/2$ and $offset = -a*(Ds/2)^2$;

We further need to select Hv and/or Dv such that the focus of the parabola coincides with the location of the light source at [x = $Dv/2$, y = 0], which is achieved by choosing:

$$Hv = ((Dv/2)^2 - (Ds/2)^2) / Ds$$

The resulting optical element is near optimal given the physical constraints of the system. Following the etendue conservation principle, the amount of collimation is proportional to $(Dv/Ds)^2$, with higher design volumes resulting is greater collimation. The cut-off angle of this optical element is given by:

$$Theta = +/- atan( (Dv/2 + Ds/2) / Hv )$$

Suitable broadband UVC light sources for use include any of a low pressure mercury lamp, a medium pressure mercury lamp, a xenon arc lamp, or an excimer lamp. Suitable low pressure mercury lamps include those commercially available from Heraeus-Noblelight (Hanau, Germany), including low pressure mercury amalgam lamps. For instance, a low pressure mercury lamp can provide a peak emission at approximately 254 nm and minimal emission at wavelengths about 245 nm and below as well as about 260 nm and above. Suitable medium pressure mercury lamps include those commercially available from Helios Quartz Americas (Sylvania, OH). Employing a Type 214 quartz sleeve or a synthetic quartz sleeve with a medium pressure mercury lamp can increase the amount of emission at 200 nm to 51% or 89%, respectively. Although the peak emission of medium pressure mercury lamps is at approximately 320 nm, medium pressure mercury lamps are polychromatic and also have several significant emission peaks between about 245 nm and about 300 nm, for instance at approximately 265 nm, as well as a broad emission band between about 210 nm and about 240 nm.

Suitable xenon arc lamps are commercially available from Atlas Material Testing Technology, Inc., (Chicago, IL), Newport (Irvine, CA), and Xenex (San Antonio, TX). Xenon arc lamps tend to have broad emission spectra starting somewhere between about 200 nm and 250 nm, and extending beyond 800 nm, with some minor peaks at about 475 nm and about 775 nm.

Examples of excimer ultraviolet light sources include lamps such as those commercially available from Osram (Massachusetts, United States), Heraeus-Noblelight (Hanau, Germany), Ushio (Tokyo, Japan), and those described in Kogelschatz, Applied Surface Science, 54 (1992), 410-423, glow discharge lamps such as those described in EP Patent Appl. 521,553 (assigned to N. V. Philips), deuterium lamps available from Hamamatsu (Hamamatsu City, Japan), microwave driven lamps such as those described in Kitamura et al, Applied Surface Science, 79/80 (1994), 507-513 and DE 4302555 AI (assigned to Fusion Systems), and excimer lamps pumped by a volume discharge with ultraviolet preionization as described in Tech. Phys, 39(10), 1054 (1994). Excimer ultraviolet light sources often comprise krypton bromide or krypton chloride. For instance, a deuterium lamp typically has emission spectra showing a broad peak bandwidth between about 200 nm and about 280 nm, then tailing off between about 280 nm to about 700 nm.

Some additional examples of different configurations of devices according to the present disclosure are illustrated schematically in FIGS. 6A, 7A, 8A, and 9:

FIG. 6A is a schematic cross-sectional view of the modeled exemplary device of Example 2 (described below). The device 600 of FIG. 6A includes a housing 602, a portion of which forms a parabolic shape, and a multilayer article 606 attached to at least a portion of the parabolic shape of the housing 602. The device 600 further includes a broadband UVC light source 604, a window 608, and a concave linearly continuous film angular control element 609 disposed between the window 608 and the broadband UV light source 604.

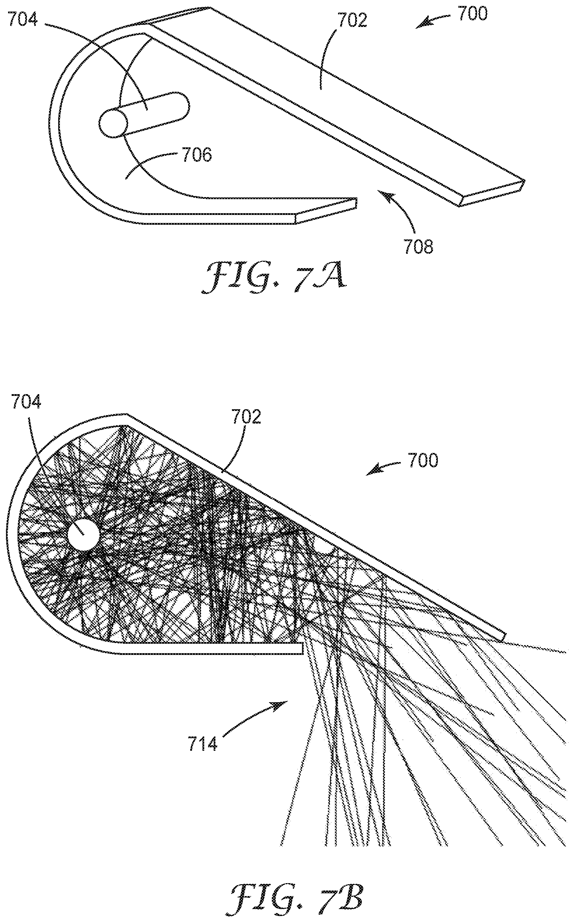

FIG. 7A is a schematic cross-sectional view of the modeled exemplary device of Example 3 (described below). The device 700 of FIG. 7A includes a housing 702, a portion of which forms the shape of a hemisphere, and a multilayer article 706 attached to at least a portion of the hemisphere of the housing 702. The device 700 further includes a broadband UVC light source 704 disposed in the housing 702 and a window 708.

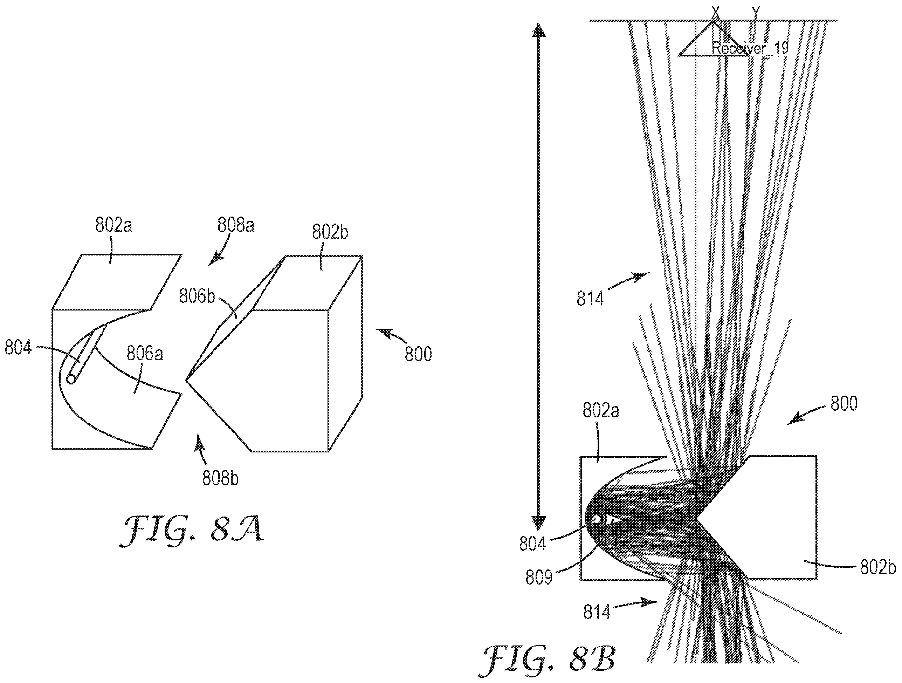

FIG. 8A is a schematic cross-sectional view of the modeled exemplary device of Example 4 (described below). The device 800 of FIG. 8A includes a first section of a housing 802a, a portion of which forms a parabolic shape, and a multilayer article 806a attached to at least a portion of the hemisphere of the housing 802a. The device 800 further includes a broadband UVC light source 804 disposed in the first section of the housing 802a. The device 800 also has a second section of a housing 802b, a portion of which forms a triangular shape, and a multilayer article 806b attached to at least a portion of the triangular shape of the housing 802b. The second section of the housing 802b is spaced apart from the first section of the housing 802a, and a point of the triangular shape of the second section of the housing 802b is positioned opposite an apex of the parabolic shape of the first section of the housing 802a. In this embodiment, the device 800 defines two windows 808a and 808b, defined by the spacing between two faces of the triangular shape of the housing 802b and the parabolic shape of the housing 802a. The windows 808a and 808b allow ultraviolet radiation (814, shown in FIG. 8B) to travel out of the device 800 in opposing directions.

*Methods*

In a second aspect, the present disclosure provides a method of disinfecting at least one material. The method comprises:

a) obtaining a device according to the first aspect;

b) directing ultraviolet radiation emitted by the broadband UVC light source through the window; and

c) exposing the at least one material to the ultraviolet radiation passing through the window for a time sufficient to achieve a desired degree of disinfection of the at least one material, optionally until achievement of a log 2, log 3, log 4, or greater reduction in an amount of at least one microorganism present on or in the at least one material, as compared to an amount of the at least one microorganism present prior to exposing the at least one material to the ultraviolet radiation passing through the window.

The device is according to any of the embodiments of the first aspect, described in detail above.

The UV reflection tends to minimize absorption of the desired wavelengths of UVC light before it is absorbed by the microorganisms it is intended for. Some exemplary materials that could be disinfected using the exemplary devices include for instance, surfaces (e.g., walls, floors, ceilings, tabletops, countertops, etc.), furniture, doorknobs/handles, light switches, electronic devices, medical instruments, hygiene articles, air, liquids (e.g., water or beverages), filter media, food preparation devices (e.g., a surface, a cutting device, a mixing device, or a cooking device), and porous membranes.

In certain embodiments, step c) above is performed until achievement of a log 2, log 3, log 4, or greater reduction of at least one microorganism on or in the at least one material, as compared to an amount of the at least one microorganism present prior to step c). As used herein, the term "microorganism" refers to any cell or particle having genetic material suitable for analysis or detection (including, for example, bacteria, yeasts, viruses, and bacterial endospores). Log reduction values (LRV) may be determined by measuring the number of colonies of a microorganism present on or in a material prior to disinfection via an exemplary method, disinfecting the material using the method, measuring the number of colonies present on or in the material following disinfection, then calculating the LRV based on colony counts obtained. The method of measuring the number of colony forming units (cfus) on or in a material will vary based on the form of the particular material. For instance, a solid may be swabbed, and a liquid or gas volumetrically sampled (and concentrated if necessary). The cfus may be measured, for instance, using a culture-based method, an imaging detection method, a fluorescence-based detection method, a colorimetric detection method, an immunological detection method, a genetic detection method, or a bioluminescence-based detection method. The LRV is then calculated using the formula below:

LRV= (Log of cfus/area or volume of pre-disinfected material) - (Log of cfus/area or volume of disinfected material)

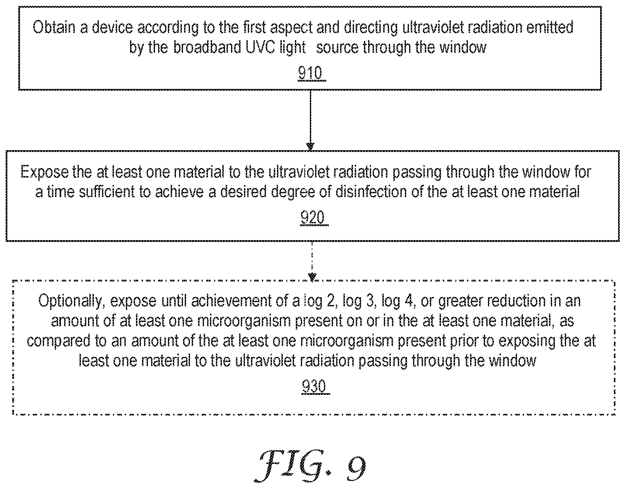

FIG. 9 provides a flow chart of an exemplary method, including Step 910 to obtain a device according to the first aspect and directing ultraviolet radiation emitted by the broadband UVC light source through the window; and Step 920 to expose at least one material to ultraviolet radiation passing through the window for a time sufficient to achieve a desired degree of disinfection of the at least one material. Generally, the at least one material comprises at least one of a solid, a liquid, or a gas. As discussed above, in some cases, it is preferable to expose the material(s) to ultraviolet radiation (e.g., light) having wavelengths of 195 nm or greater or 200 nm or greater, to 230 nm, 235 nm, or 240 nm. The method further optionally includes Step 930 to expose until achievement of a log 2, log 3, log 4, or greater reduction in an amount of at least one microorganism present on or in the at least one material, as compared to an amount of the at least one microorganism present prior to exposing the at least one material to the ultraviolet radiation passing through the window.

In preferred embodiments, during the method the one or more materials is exposed to 10, 8, 6, 5, 4, 3, 2, or 1 percent or less of ultraviolet light having a wavelength of greater than 230 nanometers, 235 nm, or 240 nm, to 400 nanometers that is emitted by the broadband UVC source. This is accomplished by effective absorption of those wavelengths by the absorbent layer, such that 90 percent or more of ultraviolet light having a wavelength of greater than 230 nanometers, 235 nm, or 240 nm, to 400 nanometers is absorbed instead of directed at and/or reflected towards the material(s) during the method.

*Systems*

In a third aspect, the present disclosure provides a system. The system comprises:

a) a device according to the first aspect;

b) one or more electronic sensors configured to generate data that is indicative of an operation of the device; and

c) at least one computing device comprising one or more computer processors and a memory comprising instructions that when executed by the one or more computer processors cause the one or more computer processors to: receive the data that is indicative of the operation of the device; apply the data to a disinfection model stored in the memory, the disinfection model characterizing the activity of the device; and wirelessly send to the device feedback that is based on the disinfection model, based on the identification of device activity.

In a fourth aspect the present disclosure provides a computing device. The computing device comprises:

a) one or more computer processors; and

b) a memory comprising instructions that when executed by the one or more computer processors cause the one or more computer processors to: obtain usage data from a device of the first aspect, wherein the usage data comprises data indicative of operation of the device; apply the usage data to a disinfection model stored in the memory, the disinfection model characterizing the activity of the device; and wirelessly send to the device feedback that is based on the disinfection model, based on the identification of device activity.

The third and fourth aspects are each described below.

The device is according to any of the embodiments of the device of the first aspect, described in detail above.

Usage data can include, for instance and without limitation, data from a UVC dosimeter that measures what dosage of UVC light has been emitted from a device and an amount of time that a device has been in use. Exemplary useful dosimeters include for instance, Model# ILT2400-UVC available from International Light Technologies, Inc. (Peabody, MA, USA); Thorlabs Model# PM100D with S120VC sensor available from Thorlabs Inc. (Newton, New Jersey, USA); and Model# X1-1-UV-3727 available from GigaHertz-Optik, Inc. (Türkenfeld, Deutschland). In some cases, a material subjected to disinfection by the device is tested for microorganisms and the test results are wirelessly sent to the computer processor(s) for incorporation into the disinfection model.

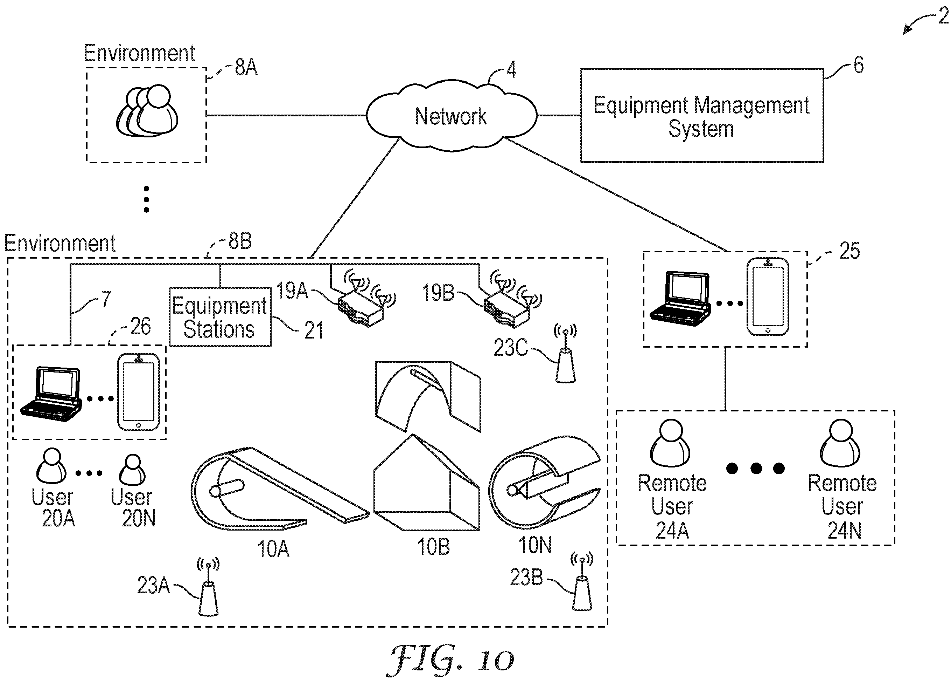

FIG. 10 is a block diagram illustrating an exemplary system 2 in which devices having communication capabilities are utilized and managed, according to aspects of this disclosure. System 2 includes equipment management system (EMS) 6, which is configured to provide device management functionalities with respect to disinfection devices 10A-10N in accordance with aspects of this disclosure. As described herein, EMS 6 enables authorized users to perform disinfection actions and manage use and maintenance of disinfection devices 10A-10N. By interacting with EMS 6, managing professionals can, for example, manage device usage, disinfection data, and area inspections. In general, EMS 6 provides data acquisition, monitoring, activity logging, reporting, predictive analytics, feedback, and alert generation.

For example, EMS 6 includes an underlying analytics and disinfection event prediction engine and alerting system in accordance with various examples described herein. As further described below, EMS 6 provides an integrated suite of disinfection equipment management tools and implements various techniques of this disclosure. That is, EMS 6 provides an integrated, end-to-end system for managing disinfection equipment, e.g., disinfection devices, used by users 20A-20N within one or more physical environments 8, which may be public transportation sites (e.g., an airport, plane, transportation station, train, bus, car, etc.), manufacturing sites, medical sites (e.g., a clinic, hospital, etc.), office sites, retail sites, education sites, food preparation sites, or any other type of physical environment. The techniques of this disclosure may be realized within various parts of system 2.

As shown in the example of FIG. 1, system 2 represents a computing environment in which a computing device within one of a plurality of physical environments 8A, 8B (collectively, "physical environments 8") electronically communicate with EMS 6 via one or more computer networks 4. Each respective physical environment 8 represents a physical environment in which one or more individuals, such as users 20A-N, utilize disinfection equipment while engaging in tasks or activities within the respective physical environment 8. In this example, physical environment 8A is shown as generally as having one or more users generally present, while physical environment 8B is shown in expanded form to provide a more detailed example. In the example of FIG. 10, a plurality of users 20A-20N are shown as having disinfection devices 10A, 10B, and 10N for their use, which are shown in this example as the devices of FIGS. 7A, 8A, and 5A, respectively (each as described in detail above).

In some examples, one or more of disinfection devices 10A-10N may include embedded sensors or monitoring devices and processing electronics configured to capture data in real-time as a worker (e.g., one of users 20A-20N) engages in activities involving disinfection devices 10A-10N. For example, disinfection devices 10A-10N may include a variety of sensor hardware such as one or more of a dosimeter, a usage timer, a location sensor, one or more environment sensors, and/or other sensors for measuring operations of disinfection devices 10A-10N. In some examples, one or more of disinfection devices 10A-10N may include data output hardware for outputting data indicative of the operation of disinfection devices 10A-10N and/or generating and outputting communications to the respective user 20 utilizing respective disinfection devices 10A-10N.

In various examples, one or more of disinfection devices 10A-10N may include or be coupled to hardware configured to render and output audible feedback (e.g., renderer(s) coupled with one or more speakers), visual feedback (e.g., video card(s) coupled with one or more displays, light emitting diodes (LEDs) or the like), or tactile feedback (e.g., a device that vibrates or provides other haptic feedback). In general, each of physical environments 8 may include computing facilities (e.g., a local area network or LAN) via which disinfection devices 10A-10N can communicate (e.g., using relay capabilities provided by network 4) with EMS 6. For example, physical environments 8 may be configured with wireless technology, such as wireless network that includes connections defined by the Institute of Electrical and Electronics Engineers (IEEE) 802.11 family of protocols, ZigBee® network connections (conforming to the IEEE 802.15 family of standards), 5G® network connections, short-range wireless (e.g., Bluetooth® and/or near-field communication (NFC)) connections, or the like. Physical environments 8 may also include or interface to wired networks, such as via Ethernet® connections that provide access to network 4.

In the example of FIG. 10, physical environment 8B includes a local network 7 that provides a packet-switched network for communicating with EMS 6 via network 4. In addition, physical environment 8B includes a plurality of wireless access points 19A, 19B that may be positionally distributed throughout physical environment 8B to provide support for wireless communications throughout physical environment 8B. One or more of disinfection devices 10A-10N is configured to communicate data, such as sensed motions, events, and conditions, via wireless communications, such as via wireless and/or wired communication protocols. One or more of disinfection devices 10A-10N may, for example, communicate directly with wireless access point 19A or 19B.

As shown in the example of FIG. 10, an environment, such as physical environment 8B, may also include one or more wireless-enabled beacons, such as beacons 23A-23C, that provide accurate location information within the environment. For example, beacons 23 may be enabled with Global Positioning System (GPS) or other location-tracking capabilities, such that a controller within the respective beacon 23 may be able to precisely determine the position of the respective beacon 23. Based on wireless communications with one or more of beacons 23, a given disinfection device 10A-10N or communication may determine the location of a user 20 within physical environment 8B. In this way, event data reported to EMS 6 may be stamped with positional information to aid analysis, reporting and analytics performed by EMS 6. In addition, an environment, such as environment 8B, may also include one or more wireless-enabled equipment stations, such as equipment stations 21. Equipment stations 21 may include one or more sensors and a controller configured to output data indicative of sensed device usage. Equipment stations 21 may be positioned within respective geographic regions of environment 8B or may otherwise interact with beacons 23 to determine respective positions and include such positional information when reporting environmental data to EMS 6. As such, EMS 6 may be configured to correlate usage data for disinfection devices 10A-10N including time, dosage, location, etc., as well as microorganism data.

While disinfection devices 10A-10N may typically transmit usage data from sensors of disinfection devices 10A-10N to EMS 6 over network 4, in some use case scenarios, disinfection devices 10A-10N may not have connectivity to network 4. In such instances, disinfection devices 10A-10N may store usage data locally and transmit the usage data to equipment stations 21. Equipment stations 21 may then upload the data received from disinfection devices 10A-10N to EMS 6 over network 4. In addition, each of physical environments 8 include computing facilities that provide an operating environment for end-user computing devices 26 for interacting with EMS 6 via network 4. One or more of users 20 may interact with computing devices 26 to access EMS 6. Similarly, remote users 24A-24N may use computing devices 25 to interact with EMS 6 via network 4. For example, the end-user computing devices 25 may include, be, or be part of laptops, desktop computers, mobile devices such as tablet computers or so-called "smartphones," and the like.

Users 20, 24 (e.g., 20A-20N, 20A-24N) interact with EMS 6 to control and actively manage many aspects of disinfection devices 10A-10N, such as accessing and viewing usage records, analytics, and reporting. For example, users 20, 24 may review usage information acquired and stored by EMS 6, where the usage information may include data specifying starting and ending times over a time duration (e.g., a day, a week, or the like), data collected during particular events, such as applied dosage, sensed data acquired from the user, microorganism data, environment data, and the like. In addition, users 20, 24 may interact with EMS 6 to perform asset tracking and to schedule maintenance events for individual pieces of disinfection equipment, e.g., disinfection devices 10A-10N, to ensure compliance with any procedures or regulations. EMS 6 may enable users 20, 24 to create and complete digital checklists with respect to the maintenance procedures and to synchronize any results of the procedures from computing devices 25, 26 to EMS 6. Further, as described herein, EMS 6 integrates an event processing platform configured to process potentially thousands or even millions of concurrent streams of events from digitally enabled disinfection equipment, such as disinfection devices 10A-10N. An underlying analytics engine of EMS 6 applies historical data and models to the inbound streams to compute assertions, such as identified anomalies or predicted occurrences of disinfection quality based on conditions or behavior patterns of users 20, 24. Further, EMS 6 provides real-time alerting and reporting to notify users 20, 24 of any predicted events, anomalies, trends, and the like.

The analytics engine of EMS 6 may, in some examples, apply analytics to identify relationships or correlations between sensed user data, microorganism data, environmental conditions, geographic regions, and other factors, and analyze the impact on disinfection events. EMS 6 may determine, based on the data acquired across physical environments 8, which particular activities lead to, or are predicted to lead to, unusually high occurrences of microorganism presence requiring disinfection. In this way, EMS 6 tightly integrates comprehensive tools for managing disinfection devices 10A-10N with an underlying analytics engine and communication system to provide data acquisition, monitoring, activity logging, reporting, behavior analytics, and alert generation. Moreover, EMS 6 provides a communication system for operation and utilization by and between the various elements of system 2.

Users 20, 24 may access EMS 6 to view results on any analytics performed by EMS 6 on data acquired from environments 8. In some examples, EMS 6 may present a web-based interface via a web server (e.g., an HTTP server) or client-side applications may be deployed for devices of computing devices 25, 26 used by users 20, 24, such as desktop computers, laptop computers, mobile devices such as smartphones or tablets, or the like. In some examples, EMS 6 may provide a database query engine for directly querying EMS 6 to view acquired device use, disinfection information, and/or any results of the analytic engine, e.g., by the way of dashboards, alert notifications, reports and the like. That is, users 20, 24, or software executing on computing devices 25, 26, may submit queries to EMS 6 and receive data corresponding to the queries for presentation in the form of one or more reports or dashboards. Such dashboards may provide various insights regarding system 2, such as baseline ("normal") operation across physical environments 8, identifications of any anomalous workers engaging in abnormal disinfection device use activities that may potentially result in inadequate disinfection, identifications of any geographic regions within environments 8A-8N for which unusually anomalous (e.g., high) microorganism presence have been or are predicted to occur, and the like.

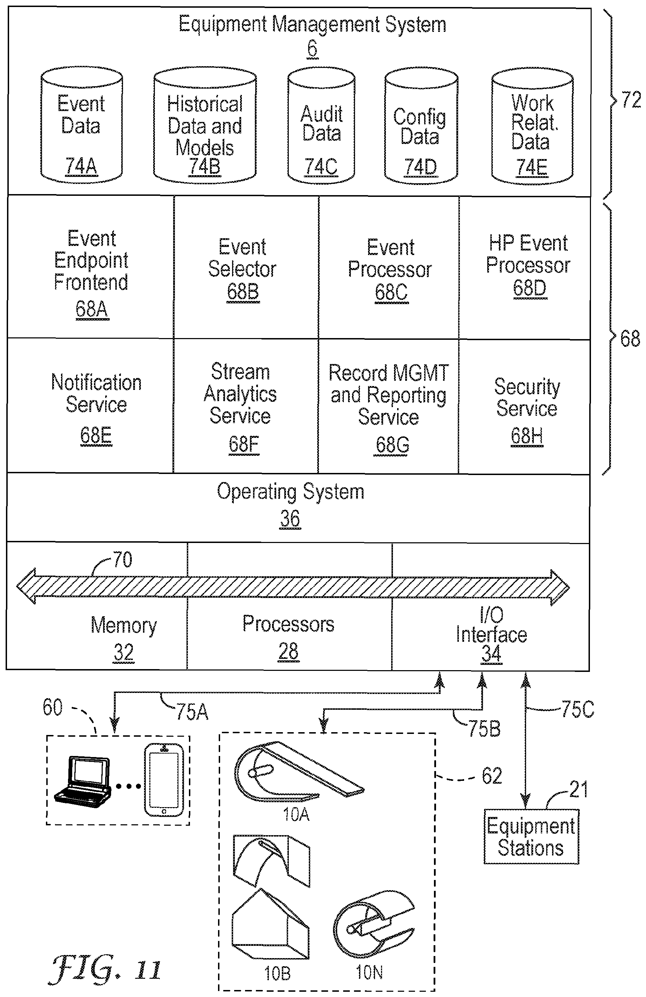

FIG. 11 is a block diagram illustrating an operating perspective of one example implementation of equipment management system (EMS) 6 shown in FIG. 10. While FIG. 11 shows one implementation of EMS 6 that is consistent with aspects of this disclosure, it will be appreciated that other architectures (whether single-device or distributed architectures) of EMS 6 are consistent with aspects of this disclosure, as well.

In the example of FIG. 11, EMS 6 includes one or more processors 28 and memory 32. In some examples, memory 32 and processors 28 may be integrated into a single hardware unit, such as a system on a chip (SoC) or integrated circuit (IC). Each of processors 28 may comprise one or more of a multi-core processor, a controller, a digital signal processor (DSP), an application specific integrated circuit (ASIC), a field-programmable gate array (FPGA), processing circuitry (e.g., fixed function circuitry, programmable circuitry, or any combination of fixed function circuitry and programmable circuitry) or equivalent discrete logic circuitry or integrated logic circuitry. Memory 32 may include any form of memory for storing data and executable software instructions, such as random-access memory (RAM), read-only memory (ROM), programmable read only memory (PROM), erasable programmable read-only memory (EPROM), electronically erasable programmable read only memory (EEPROM), and flash memory.

Memory 32 and processor(s) 28 provide a computer platform for executing operation system 36. In turn, operating system 36 provides a multitasking operating environment for executing one or more software components 68. As shown, processors 28 connect via an input/output (I/O) interface 34 to external systems and devices, such as to interfaces deployed at equipment stations 21, computing devices 60, disinfection devices 62, and the like. I/O interface 34 may incorporate network interface hardware, such as one or more wired and/or wireless network interface controllers (NICs) for communicating via communication channels 75A-75C, which may represent one or more network-enabled communicative connections, such as one or more packet-switched networks. Bus 70 provides inter-component connectivity between processors 28, memory 32, and I/O interface 34 in the implementation shown in FIG. 11. Bus 70 may represent a half-duplex or full-duplex bus that provides data transfer capabilities between two or more of processors 28, memory 32, I/O interface 34, and/or any other hardware components of EMS 6. Bus 70 may represent a system bus or a computer bus of various types, including one or more bus networks. Regardless of the topology implemented, bus 70 may, in various examples, incorporate various types of inter-component connectivity hardware such as those conforming to any of first generation, second generation, third generation, or fourth generation bus or bus network technology as set forth by the IEEE, and/or other bus or bus network technologies defined in developing or later-adopted standards.

Software components 68 of EMS 6, in the particular example of FIG. 11, include event endpoint frontend 68A, event selector 68B, event processor 68C, high priority (HP) event processor 68D, notification service 68E, stream analytics service 68F, record management and reporting service 68G, and security service 68H. In some example approaches, one or more of software components 68 represent executable software instructions that may take the form of one or more software applications, software packages, software libraries, hardware drivers, and/or Application Program Interfaces (APIs). Moreover, any of software components 68 may output data and/or receive data via I/O interface 34.

Aspects of memory 32 that provide non-volatile storage and/or long-term storage support local storage of data repositories 72. In the example of FIG. 11, data repositories 72 include event data 74A, historical data and models 74B, audit data 74C, configuration data 74D, and work relation data 74E. One or more of software components 68 may invoke processors 28 and memory 32 to access one or more of data repositories 72 to retrieve data for various purposes, such as comparison, processing and relaying, and/or viewing the results of a disinfection model. In some examples, software components 68 may implement read/write capabilities with respect to data repositories 72, such as to access and use information available from data repositories 72 and/or to modify information currently stored to data repositories 72. In implementations in which EMS 6 represents a distributed computing system, one or more of data repositories 72 may be positioned at a remote location from processors 28, and software components 68 may, in these implementations, access data repositories 72 using NIC hardware of I/O interface 34.

Event endpoint frontend 68A operates as a front end interface for receiving and sending communications to disinfection devices 62. Each incoming communication may, for example, carry recently captured data representing sensed conditions, usage data, dosage, or other data, generally referred to as events. Communications exchanged between the event endpoint frontend 68A and the disinfection devices 62 may be real-time or pseudo real-time depending on communication delays and continuity.

Event selector 68B operates on the communications received from disinfection devices 62 via frontend 68A and determines, based on rules or classifications, priorities associated with the incoming events. Based on the priorities, event selector 68B enqueues the events for subsequent processing by event processor 68C or high priority (HP) event processor 68D. Additional computational resources and objects may be dedicated to HP event processor 68D so as to ensure responsiveness to critical events, such as incorrect or insufficient usage of disinfection devices. Responsive to processing high priority events, HP event processor 68D may immediately invoke notification service 68E to generate alerts, instructions, warnings, or other similar messages to be output to users 20 and/or remote users 24. Events not classified as high priority are consumed and processed by event processor 68C.

In general, event processor 68C or high priority (HP) event processor 68D operate on the incoming streams of events to update event data 74A. In general, event data 74A may include all or a subset of usage data obtained from disinfection devices 62. For example, in some instances, event data 74A may include entire streams of samples of data obtained from electronic sensors of disinfection devices 62, or alternatively a subset of such data, e.g., associated with a particular time period or activity of disinfection devices 62. Event processors 68C, 68D may create, read, update, and delete event information stored in event data 74A. Event information may be stored in a respective database record as a structure that includes name/value pairs of information, such as data tables specified in row/column format. For instance, a name (e.g., column) may be "worker ID" and a value may be an employee identification number. An event record may include information such as, but not limited to: worker identification, disinfection device identification, acquisition timestamp(s) and data indicative of one or more sensed parameters. Additionally, event processors 68C, 68D may create, read, update, and delete a disinfection model generated from usage data (e.g., for disinfection devices 10A-10N) including time, dosage, location, etc., as well as microorganism data.

In addition, event selector 68B directs the incoming stream of events to stream analytics service 68F, which represents an example of an analytics engine configured to perform in depth processing of the incoming stream of events to perform real-time analytics. Stream analytics service 68F may, for example, be configured to process and compare multiple streams of event data 74A with historical data and models 74B in real-time as event data 74A is received. In this way, stream analytic service 68D may be configured to detect anomalies, transform incoming event data values, trigger alerts upon detecting disinfection concerns based on conditions or worker behaviors. Historical data and models 74B may include, for example, specified disinfection rules, business rules, safety rules, and the like. In this way, historical data and models 74B may characterize activity of a user 20, e.g., as conforming to the rules. In addition, stream analytic service 68D may generate output for communicating to disinfection devices 62 by stream analytics service 68F or computing devices 60 by way of record management and reporting service 68D.

In this way, analytics service 68F processes inbound streams of events, potentially hundreds or thousands of streams of events, from enabled disinfection devices 62 utilized by users 20 within environments 8 to apply historical data and models 74B to compute assertions, such as identified anomalies or predicted occurrences of disinfection quality based on conditions or behavior patterns of users 20. Stream analytics service 68F may publish the assertions to notification service 68E and/or record management and reporting service 68G based on processing information stored by EMS 6 to provide actionable information to any of computing devices 60.

Record management and reporting service 68G processes and responds to messages and queries received from computing devices 60 via communication connection 75A. For example, record management and reporting service 68G may receive requests from client computing devices for event data related to individual users, populations or sample sets of users, geographic regions of environments 8 or environments 8 as a whole, individual or groups/types of disinfection devices 62. In response, record management and reporting service 68G accesses event information based on the request. Upon retrieving the event data, record management and reporting service 68G constructs an output response to the client application that initially requested the information. In some examples, the data may be included in a document, such as an HTML document, or the data may be encoded in a JSON format or presented by a dashboard application executing on the requesting client computing device. For instance, as further described in this disclosure, example user interfaces that include the event information are depicted in the figures. As additional examples, record management and reporting service 68G may receive requests to find, analyze, and correlate disinfection event information. For instance, record management and reporting service 68G may receive a query request from a client application for event data 74A over a historical time frame, such as a user can view disinfection event information over a period of time and/or a computing device can analyze the disinfection event information over the period of time.

In example implementations, services 68 may also include security service 68H that authenticate and authorize users and requests with EMS 6. Specifically, security service 68H may receive authentication requests from client applications and/or other services 68 to access data in data repository 72. An authentication request may include credentials, such as a username and password. Security service 68H may query configuration data 74D to determine whether the username and password combination is valid. Configuration data 74D may include security data in the form of authorization credentials, policies, and any other information for controlling access to EMS 6. Other credentials may include device identifiers or device profiles that are allowed to access EMS 6. Security service 68H may provide audit and logging functionality for operations performed at EMS 6. For instance, security service 68H may log operations performed by services 68 and/or data accessed by services 68 in data repository 72. Security service 68H may store audit information such as logged operations, accessed data, and rule processing results in audit data 74C. In some examples, security service 68H may generate events in response to one or more rules being satisfied. Security service 68H may store data indicating the events in audit data 74C.

Exemplary Embodiments

In a first embodiment, the present disclosure provides a device. The device comprises a) a housing that is substantially impermeable to ultraviolet radiation having a wavelength of from 280 nm to 400 nm, and at least one window defined in the housing; b) a broadband UVC light source positioned within the housing or within an optional enclosure adjacent to the housing; and c) a multilayer article positioned within the housing. The broadband UVC light source is configured to direct light at the ultraviolet mirror of the multilayer article. The multilayer article comprises: i) an absorbent layer that absorbs, scatters, or both, at least 50, 60, 70, 80, 90, or 95 percent of incident ultraviolet light having a wavelength between at least 230 nanometers and 400 nanometers. The absorbent layer has a major surface. The multilayer article further comprises ii) an ultraviolet mirror adjacent to the major surface of the absorbent layer, wherein the ultraviolet mirror is comprised of at least a plurality of alternating first and second optical layers collectively reflecting at an incident light angle of at least one of 0°, 15°, 30°, 45°, 60°, or 75°, at least 50, 60, 70, 80, 90, or 95 percent of incident ultraviolet light in a wavelength range from 195 nanometers or 200 nm, to 230 nanometers, 235 nm, or 240 nm, and collectively transmitting at an incident light angle of at least one of 0°, 15°, 30°, 45°, 60°, or 75°, at least 50, 60, 70, 80, 90, or 95 percent of incident ultraviolet light in a wavelength range from greater than 230 nanometers, greater than 235 nm, or greater than 240 nm, to 400 nanometers.

In a second embodiment, the present disclosure provides a device according to the first embodiment, wherein the housing is configured to block direct passage of light from the broadband UVC light source through the window, with the exception of light that travels a distance of at least 3 centimeters (cm), 3.25 cm, 3.5 cm, 3.75 cm, or at least 4 cm from the broadband UVC light source to the window.

In a third embodiment, the present disclosure provides a device according to the first embodiment, wherein the housing is configured to block direct passage of light from the broadband UVC light source through the window.

In a fourth embodiment, the present disclosure provides a device according to any of the first through third embodiments, wherein a cross-section of at least a portion of the housing forms a semicircular shape, a circular shape, an elliptical shape, a parabolic shape, or a rectangular shape.

In a fifth embodiment, the present disclosure provides a device according to any of the first through fourth embodiments, wherein a cross-section of at least a portion of the housing forms a semicircular shape.

In a sixth embodiment, the present disclosure provides a device according to any of the first through fourth embodiments, wherein a cross-section of at least a portion of the housing forms a circular shape.

In a seventh embodiment, the present disclosure provides a device according to any of the first through fourth embodiments, wherein a cross-section of at least a portion of the housing forms a parabolic shape.

**In** an eighth embodiment, the present disclosure provides a device according to any of the first through fourth embodiments, wherein a cross-section of at least a portion of the housing forms a rectangular shape.

In a ninth embodiment, the present disclosure provides a device according to any of the first through eighth embodiments, further comprising at least one angular control element disposed in the housing.

In a tenth embodiment, the present disclosure provides a device according to the ninth embodiment, wherein the at least one angular control element comprises a film.

In an eleventh embodiment, the present disclosure provides a device according to the ninth embodiment or the tenth embodiment, wherein the at least one angular control element comprises a solid structure.

In a twelfth embodiment, the present disclosure provides a device according to the ninth embodiment or the tenth embodiment, wherein the at least one angular control element comprises a hollow structure.

In a thirteenth embodiment, the present disclosure provides a device according to any of the ninth through twelfth embodiments, wherein the at least one angular control element has rotational symmetry.

In a fourteenth embodiment, the present disclosure provides a device according to any of the ninth through twelfth embodiments, wherein the at least one angular control element is linearly continuous.

In a fifteenth embodiment, the present disclosure provides a device according to the tenth embodiment, wherein the at least one angular control element comprises a concave shape.

In a sixteenth embodiment, the present disclosure provides a device according to the fifteenth embodiment, wherein the broadband UVC light source is disposed between a first concave shape formed by the housing and a second concave shape formed by the angular control element, wherein the first concave shape is curved in an opposing direction than the second concave shape.

In a seventeenth embodiment, the present disclosure provides a device according to any of the ninth through sixteenth embodiments, wherein the at least one angular control element comprises at least one of a collimator, a retroreflector, a diffuser, or a reflecting diverter.

In an eighteenth embodiment, the present disclosure provides a device according to the tenth embodiment, wherein the at least one angular control film comprises a lens.

In a nineteenth embodiment, the present disclosure provides a device according to any of the ninth through eighteenth embodiments, wherein the at least one angular control film is disposed between the broadband UVC light source and the window.

In a twentieth embodiment, the present disclosure provides a device according to any of the ninth through nineteenth embodiments, wherein the at least one angular control film comprises a fluoropolymer.

In a twenty-first embodiment, the present disclosure provides a device according to any of the first through twentieth embodiments, wherein the housing further defines a second window.

In a twenty-second embodiment, the present disclosure provides a device according to any of the first through twenty-first embodiments, wherein the broadband UVC light source is a low pressure mercury lamp, a medium pressure mercury lamp, a xenon arc lamp, a deuterium arc lamp, a germicidal lamp, or an excimer lamp.

In a twenty-third embodiment, the present disclosure provides a device according to any of the first through twenty-second embodiments, further comprising an adhesive layer adjacent to the absorbent layer, wherein the absorbent layer is disposed between the ultraviolet mirror and the adhesive layer.

In a twenty-fourth embodiment, the present disclosure provides a device according to any of the first through twenty-third embodiments, wherein the absorbent layer absorbs at least 30 percent, at least 40 percent, at least 50 percent, at least 60 percent, at least 70 percent, at least 80 percent, or at least 90 percent of incident visible light having a wavelength between at least 400 nanometers and 700 nanometers.

In a twenty-fifth embodiment, the present disclosure provides a device according to any of the first through twenty-fourth embodiments, wherein the absorbent layer comprises a silicone thermoplastic, a fluoropolymer, copolymers thereof, or blends thereof.

In a twenty-sixth embodiment, the present disclosure provides a device according to any of the first through twenty-fifth embodiments, wherein the absorbent layer comprises a fluoropolymer (co)polymer comprising polymerized units derived from one or more monomers selected from tetrafluoroethylene, hexafluoropropylene, vinylidene fluoride, a perfluoroalkoxy alkane, or a combination thereof.

In a twenty-seventh embodiment, the present disclosure provides a device according to any of the first through twenty-sixth embodiments, wherein the absorbent layer further comprises one or more of an ultraviolet radiation absorber, an ultraviolet radiation scatterer, a hindered amine light stabilizer, an anti-oxidant, a pigment, or a combination thereof.

In a twenty-eighth embodiment, the present disclosure provides a device according to the twenty-seventh embodiment, wherein the ultraviolet radiation absorber comprises at least one of carbon black, titanium dioxide, zinc oxide, cesium dioxide, aluminum oxide, or zirconium dioxide.

In a twenty-ninth embodiment, the present disclosure provides a device according to the twenty-seventh embodiment of the twenty-eighth embodiment, wherein the ultraviolet radiation absorber is selected from a benzotriazole compound, a benzophenone compound, a triazine compound, or a combination thereof.

In a thirtieth embodiment, the present disclosure provides a device according to any of the first through twenty-ninth embodiments, wherein the absorbent layer comprises a continuous metal coating or layer.

In a thirty-first embodiment, the present disclosure provides a device according to any of the first through thirtieth embodiments, wherein the absorbent layer comprises metal particles disposed in a polymer matrix.

In a thirty-second embodiment, the present disclosure provides a device according to the thirtieth embodiment or the thirty-first embodiment, wherein the metal is selected from silver, gold, copper, nickel, and titanium.

In a thirty-third embodiment, the present disclosure provides a device according to any of the first through thirty-second embodiments, wherein the at least first optical layer comprises at least one of zirconium oxynitride, hafnia, alumina, magnesium oxide, yttrium oxide, lanthanum fluoride, or neodymium fluoride and wherein the second optical layer comprises at least one of silica, aluminum fluoride, magnesium fluoride, calcium fluoride, silica alumina oxide, or alumina doped silica.

In a thirty-fourth embodiment, the present disclosure provides a device according to any of the first through thirty-second embodiments, wherein the at least first optical layer comprises at least one of polyvinylidene fluoride or polyethylene tetrafluoroethylene and wherein the second optical layer comprises a copolymer of tetrafluoroethylene, hexafluoropropylene, and vinylidene fluoride.

**In** a thirty-fifth embodiment, the present disclosure provides a device according to any of the first through thirty-fourth embodiments, wherein the ultraviolet mirror absorbs at an incident light angle of at least one of 0°, 15°, 30°, 45°, 60°, or 75°, at least 30 percent, at least 80 percent, at least 90 percent, at least 95 percent, or at least 98 percent of incident visible light having a wavelength between at least 400 nanometers and 700 nanometers.

**In** a thirty-sixth embodiment, the present disclosure provides a device according to any of the first through thirty-fifth embodiments, wherein the ultraviolet mirror comprises a pigment or a dye.

In a thirty-seventh embodiment, the present disclosure provides a device according to any of the first through thirty-sixth embodiments, wherein the ultraviolet mirror reflects at an incident light angle of at least one of 0°, 15°, 30°, 45°, 60°, or 75°, at least 80 percent, at least 90 percent, at least 95 percent, or at least 98 percent of incident ultraviolet light in a wavelength range from 195 nm or 200 nm, to 230 nanometers, 235 nm, or 240 nm, preferably from 195 nm to 230 nm, from 200 nm to 240 nm, or from 200 nm to 230 nm.

In a thirty-eighth embodiment, the present disclosure provides a device according to any of the first through thirty-seventh embodiments, wherein the ultraviolet mirror is directly attached to the absorbent layer.

In a thirty-ninth embodiment, the present disclosure provides a device according to any of the first through thirty-seventh embodiments, wherein the ultraviolet mirror is separated from the absorbent layer by an air gap.

In a fortieth embodiment, the present disclosure provides a device according to any of the first through thirty-ninth embodiments, further comprising at least one of a heat transfer layer, a plurality of heat transfer fins, or a plurality of heat transfer pins, adjacent to a major surface of the absorbing layer opposite the ultraviolet mirror.

In a forty-first embodiment, the present disclosure provides a device according to any of the first through fortieth embodiments, wherein a major surface of the ultraviolet mirror comprises a plurality of nonplanar features protruding from the major surface.

In a forty-second embodiment, the present disclosure provides a device according to any of the first through forty-first embodiments, further comprising a visible light source configured to emit visible light when the UVC light source is emitting light.

In a forty-third embodiment, the present disclosure provides a device according to the forty-second embodiment, wherein the visible light source is electrically or optically powered.

In a forty-fourth embodiment, the present disclosure provides a device according to any of the first through forty-third embodiments, further comprising an activation switch that causes the UVC light source to emit light or to halt emitting light when a user engages the activation switch.

In a forty-fifth embodiment, the present disclosure provides a device according to the forty-fourth embodiment, wherein the activation switch is connected to a motion detector.

In a forty-sixth embodiment, the present disclosure provides a method of disinfecting at least one material. The method comprises: obtaining the device according to of any of the first through forty-fifth embodiments; directing ultraviolet radiation emitted by the broadband UVC light source through the window; and exposing the at least one material to the ultraviolet radiation passing through the window for a time sufficient to achieve a desired degree of disinfection of the at least one material, optionally until achievement of a log 2, log 3, log 4, or greater reduction in an amount of at least one microorganism present on or in the at least one material, as compared to an amount of the at least one microorganism present prior to exposing the at least one material to the ultraviolet radiation passing through the window.

In a forty-seventh embodiment, the present disclosure provides a system. The system comprises: a device according to the first aspect; one or more electronic sensors configured to generate data that is indicative of an operation of the device; and at least one computing device comprising one or more computer processors and a memory comprising instructions that when executed by the one or more computer processors cause the one or more computer processors to: receive the data that is indicative of the operation of the device; apply the data to a disinfection model stored in the memory, the disinfection model characterizing the activity of the device; and wirelessly send to the device feedback that is based on the disinfection model, based on the identification of device activity.

In a forty-eighth embodiment, the present disclosure provides a computing device. The computing device comprises: one or more computer processors; and a memory comprising instructions that when executed by the one or more computer processors cause the one or more computer processors to: obtain usage data from a device of the first aspect, wherein the usage data comprises data indicative of operation of the device; apply the usage data to a disinfection model stored in the memory, the disinfection model characterizing the activity of the device; and wirelessly send to the device feedback that is based on the disinfection model, based on the identification of device activity.

Advantages and embodiments of this invention are further illustrated by the following examples, but the particular materials and amounts thereof recited in these examples, as well as other conditions and details, should not be construed to unduly limit this invention. All parts and percentages are by weight unless otherwise indicated.

EXAMPLES

**Preparative Example 1**

A UVC mirror reflecting over the range of 200-240 nm was made by vapor coating an inorganic optical stack having first optical layers comprising $HfO_2$ and second optical layers comprising $SiO_2$ onto 100 micrometers (4 mil) thick fluoropolymer film (obtained under the trade designation "NOWOFLON THV 815" from Nowofol Kunststoffprodukte GmbH & Co. KG, Siegsdorf, Germany). More specifically, a thin film stack comprised of alternating layers of $HfO_2$ and $SiO_2$ and designed to have peak reflectance at 222 nm, was begun by coating layer 1 $HfO_2$ at 23.5.00 nm. In electron beam deposition, a four-hearth evaporation source was used. Each hearth was cone-shaped and contained 17 $cm^3$ volume of $HfO_2$ chunks. A magnetically deflected high voltage electron beam was raster scanned over the material surface as filament current of the beam was steadily, in a pre-programmed fashion, increased. Upon completion of the pre-programmed step, the $HfO_2$ surface was heated to evaporation temperature, about 2500 °C, a source shutter was opened, and the $HfO_2$ vapor flux emerged from the source in a cosine-shaped distribution and condensed upon the substrate material above the source. For enhancement of coating uniformity, the substrate holders rotated during deposition. Upon reaching the prescribed coating thickness (23.50 nm) the filament current was shut off, the shutter was closed, and the $HfO_2$ material cooled. For layer 2 the evaporation source was then rotated to a hearth containing chunks of $SiO_2$ and a similar pre-programmed heating process began. Here, the $SiO_2$ surface temperature was about 950 °C when the source shutter opened and, upon reaching the prescribed coating thickness (34.2 nm), the filament current was shut off, the shutter was closed, and the $SiO_2$ material cooled. This stepwise process was continued, layer by layer, until a total number of 13 layers was reached. Reflectance was measured with a spectrophotometer (obtained under the trade designation "LAMBDA 1050 UV-VIS" from Perkin-Elmer, Waltham, MA) and found to be 97.9% at 222 nm and 18.2% at 254 nm.

A (UV) absorbent film was made by extrusion compounding 30 wt.% $TiO_2$ (obtained from Americhem Co., Cuyahoga Falls, OH) with a fluoropolymer (obtained under the trade designation "3M DYNEON THV 500GZ" from 3M, St. Paul, MN) and casting into a 200 micron thick film on a chilled roll at 24 ft/min (7.32 m/min).

The UVC mirror film was then heat laminated to the absorbent fluoropolymer film in an oven at 130 °C under 5 lbs (2.27 kg) of weight on a 36 square inch (232.3 $cm^2$) film sample for 2 hours. This heat laminated UV mirror film stack (multilayer article) was measured with a spectrophotometer (LAMBDA 1050 UV-VIS) to have an average % reflection of 89.9% over the wavelength range of 200 nm to 240 nm as shown in FIG. 2.

Preparative Example 2 - prophetic

A UVC mirror film reflecting over the range 200-240 nm could be created by sputter coating an inorganic optical stack having first optical layers comprising $ZrO_xN_y$ and second optical layers comprising $SiAl_xO_y$ onto 100 microns thick fluoropolymer film (available under the trade designation "NOWOFLON THV 815" from Nowofol Kunststoffprodukte GmbH & Co. KG, Siegsdorf, Germany). A UV transparent base film can be coated in continuous roll to roll (R2R) fashion, using $ZrO_xN_y$ as the high refractive index material and $SiAl_xO_y$ as the low refractive index material. The optical design is alternating quarter wave thickness layers of the two materials tuned to have a peak reflectance at 222 nm. For $ZrO_xN_y$, with refractive index of 3.1 at 222 nm, the physical thickness target is 17.74 nm. For $SiAl_xO_y$, here sputtered from an aluminum-doped silicon sputter target, with refractive index 1.57, the target thickness is 35 nm. Layer one $ZrO_xN_y$ is DC sputtered from a pure zirconium sputter target in a gas mixture of argon, oxygen and nitrogen. Whereas argon is the primary sputtering gas, oxygen and nitrogen levels are set to achieve transparency, low absorptance and high refractive index. The film roll transport initially starts at a pre-determined speed, and the sputter source power is ramped to full operating power, followed by introduction of the reactive gases, and then achieving steady state conditions. The sputter source is orthogonal to and wider than the film which is being coated. Upon reaching the desired length of coated film the flows of reactive gases are set to zero and the target is sputtered to provide a pure Zr surface state. The film direction is next reversed and silicon (aluminum doped) is deposited using a rotary pair of sputter targets using AC frequency (40 kHz) power applied in an argon sputtering atmosphere. Upon reaching steady state, oxygen reactive gas is introduced to provide transparency and low refractive index. At the pre-determined process setting and line speed the second layer is coated over the length which was coated for the first layer. The sputter sources are orthogonal to and wider than the film being coated. After reaching the desired length of coated film the flow of reactive oxygen is removed and the target is sputtered in argon to provide a pure aluminum-doped silicon surface state. This stepwise process is continued, layer by layer, until a total number of 9 layers is reached. Resulting peak reflectance is expected to be 95% at 222 nm with a decrease to a lower reflectance of 20% at 254 nm when measured with a spectrophotometer ("LAMBDA 1050 UV-VIS"). A (UV) absorbent film can be made by extrusion compounding 30 wt.% $TiO_2$ (available from Americhem, Cuyahoga Falls, OH) with fluoropolymer "3M DYNEON THV 500GZ" and casting into a 200 micron thick film onto a chilled roll at 10 ft/min (3.05 m/min). The UVC mirror film can then be heat laminated to the absorbent fluoropolymer film in an oven at 130 °C under 5 lbs (2.27 kg) of weight on a 36 square inch

(232.3 cm$^2$) film sample for 2 hours. This heat laminated UV mirror film stack (multilayer article) would be measured with a spectrophotometer ("LAMBDA 1050 UV-VIS") and expected to have an average % reflection of 89.9% over the wavelength range of 200 nm to 240 nm.

**Example** 1 - prophetic

Commercially available ray trace software (obtained under the trade designation "LIGHTTOOLS" from Synopsys, Mountain View, CA) was used to simulate the device performance.

In the modeled device shown in FIG 5A, the diameter of the cylindrical source is 15 mm and it emits light to all directions. The diameter of the reflective cavity is 50 mm with a 40 mm opening on one side. Two ends of the cylindrical cavity are both capped with reflective mirror as well (not shown). The light source is 20 mm off-centered, positioned away from the cavity opening. The reflective diverter is a triangular prism at the center of the reflective cavity. Its cross-section is a triangle, with a 24 mm base facing the cavity opening and a 12 mm height (the dimension from the base towards the cylindrical source). Mirror reflection is used in the model. A simple example about how the light rays travel from inside of the cavity to outside is shown in FIG. 5B.

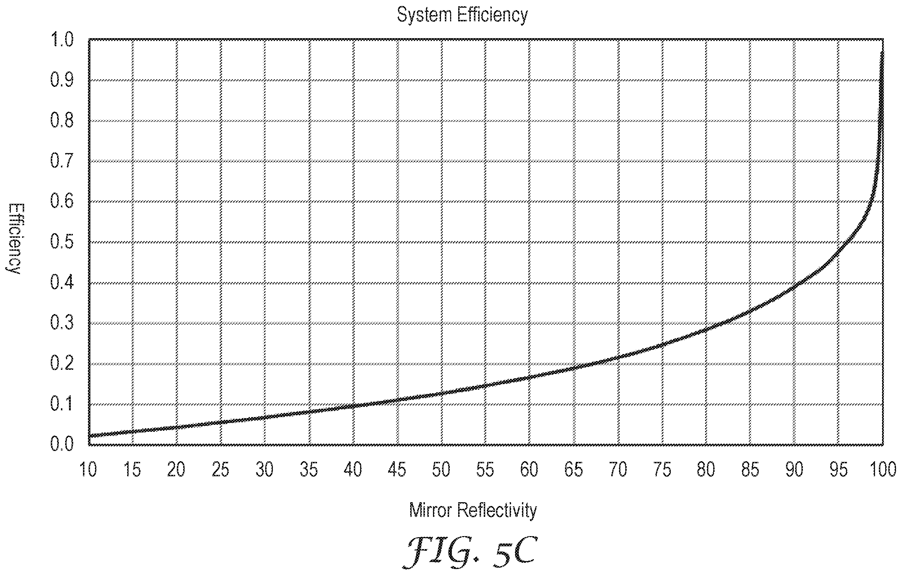

System efficiency of this device is highly dependent on the reflectivity of the cavity interior and the reflective diverter due to multiple bounces. Its dependence is simulated and shown in FIG. 5C. System efficiencies for 80% R (at 222 nm) and 20% R (at 254 nm) are approximately 28% for this example.

Example 2 - prophetic

Commercially available ray trace software (obtained under the trade designation "LIGHTTOOLS" from Synopsys, Mountain View, CA) was used to simulate the device performance.

In the modeled device shown in FIG.6A, the diameter of the source is 15 mm and it emits light to all directions. The radius of curvature of the parabolic reflector is 50 mm and its height is 250 mm. The light source is located at the focal point of the parabolic reflector. The radius of the reflective shield is 14.5 mm, located 20 mm to the right of the source, with a height of 26 mm. Two ends of the parabolic reflector are both capped with reflective mirror as well (not shown). The length of each of the parabolic reflector, the light source, and the reflective shield, is 500 mm.

A detector (500 mm x 1000 mm) is placed 1 m away from the light source. A simple example of how the light rays propagate from the source to the detector is shown in FIG. 6B.

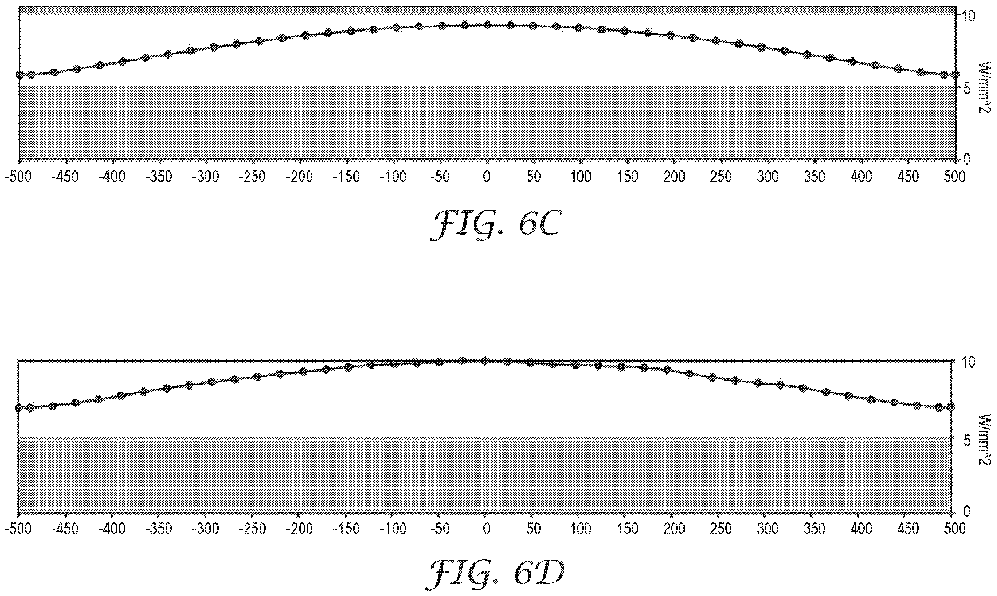

Illuminance on the detector, both magnitude and distribution, was calculated for different scenarios. As can be seen in FIG. 6C, peak illuminance of 222 nm is approximately 9 W/m$^2$ and peak illuminance of 254 nm, as can be seen in FIG. 6D, is approximately 1W/m$^2$.

**Example** 3 - **prophetic**

Commercially available ray trace software (obtained under the trade designation "LIGHTTOOLS" from Synopsys, Mountain View, CA) was used to simulate the device performance.

In the modeled device shown in FIG. 7A, the diameter of the cylindrical source is 15 mm and it emits light to all directions. The diameter of the reflective cavity is 50 mm (half circle). Two ends of the cylindrical cavity are both capped with reflective mirror (not shown). The light source is 20 mm off-centered toward the half-circle part of the reflector. The flat bottom is 100 mm long, and the flat top is 200mm long with angle of 30deg relative to horizontal direction. A simple example about how the light rays travel from inside of the cavity to outside is shown in FIG. 7B. A detector (1000 mm x 1000 mm) was placed 1 m away below the light assembly.

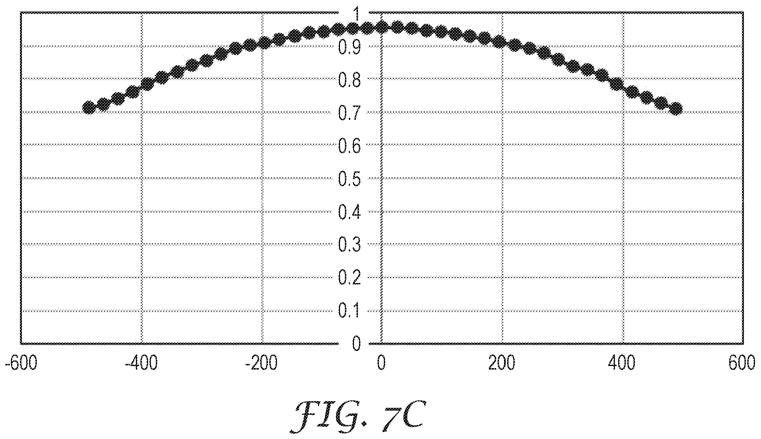

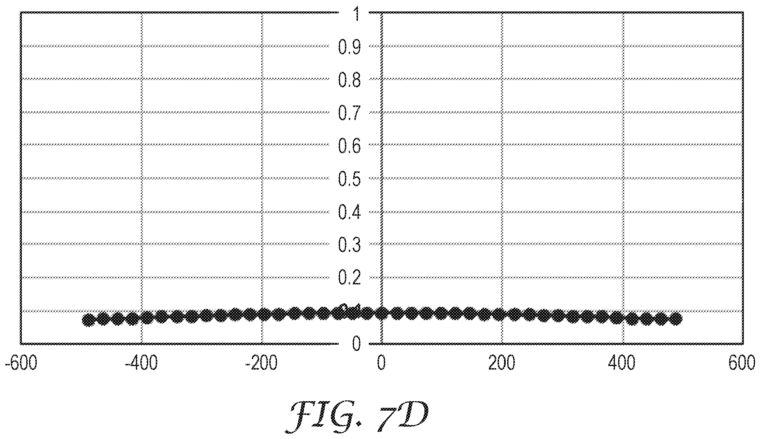

Illuminance on the detector, both magnitude and distribution, was calculated for a UVC Mirror film having a reflectivity at 222 nm of 80%. The illuminance magnitude and distribution are shown in FIG. 7C with a peak illuminance of 0.95 W/m$^2$ for 222 nm. Illuminance on the detector, both magnitude and distribution, was calculated for a UVC Mirror film having a reflectivity at 254 nm of 20%. The illuminance magnitude and distribution are shown in FIG. 7D with a peak illuminance of 0.1 W/m$^2$ for 254 nm.

**Example** 4 - **prophetic**

Commercially available ray trace software (obtained under the trade designation "LIGHTTOOLS" from Synopsys, Mountain View, CA) was used to simulate the device performance.

In the modeled device shown in FIG. 8A, the diameter of the cylindrical source is 15 mm and it emits light to all directions. The radius of curvature of the parabolic reflector is 50 mm and its height is 250 mm. The light source is located at the focal point of the parabolic reflector. The radius of the reflective shield is 14.5 mm, located 20 mm to the right of the source, with a height of 26 mm. The reflecting diverter is located 200 mm away to the right of the parabola focal point. The angle of the diverter surfaces is 48° relative to the optical axis of the parabola.

Two ends of the parabolic reflector and reflecting diverter are both capped with reflective mirror as well (not shown). The length of each of the parabolic reflector, the reflecting diverter, the light source, and the reflective shield, is 500 mm.

A detector (500 mm x 1000 mm) is placed 1 m away from the light assembly. A simple example of how the light rays propagate from the source to the detector is shown in FIG. 8B. (Another similar detector could be placed 1 m below the light assembly as well, and, due to symmetries, would give similar results.)

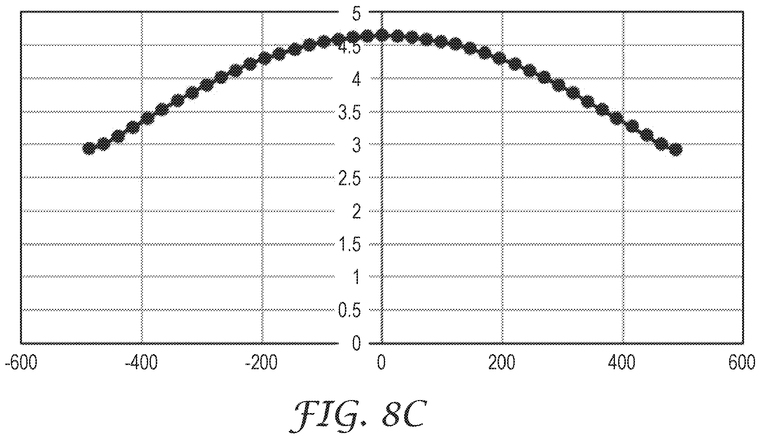

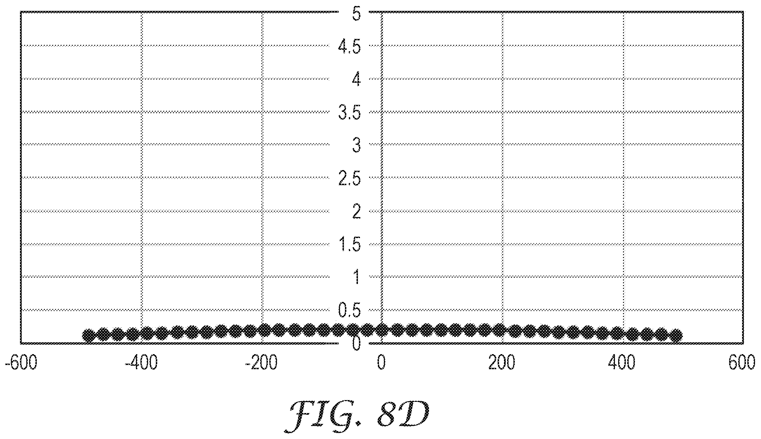

Illuminance on the detector, both magnitude and distribution, was calculated for a UVC Mirror film having a reflectivity at 222 nm of 80%. The 222 nm illuminance magnitude and distribution are shown in FIG. 8C with a peak illuminance of 4.6 W/m$^2$ for 222 nm. Illuminance on the detector, both magnitude and distribution, was calculated for a UVC Mirror film having a reflectivity at 254 nm of 20%. The illuminance magnitude and distribution are shown in FIG. 8D with a peak illuminance of 0.2 W/m$^2$ for 254 nm.

**Claims**

1. A device comprising:

   a) a housing that is substantially impermeable to ultraviolet radiation having a wavelength of from 280 nm to 400 nm, and at least one window defined in the housing;
   b) a broadband UVC light source positioned within the housing or within an optional enclosure adjacent to the housing; and
   c) a multilayer article positioned within the housing, the multilayer article comprising:

      i) an absorbent layer that absorbs at least 50, 60, 70, 80, 90, or 95 percent of incident ultraviolet light having a wavelength between at least 230 nanometers and 400 nanometers, the absorbent layer comprising a major surface; and
      ii) an ultraviolet mirror having an outer major surface and inner major surface, the inner major surface being adjacent to the major surface of the absorbent layer, wherein the ultraviolet mirror is comprised of at least a plurality of alternating first and second optical layers collectively reflecting at an incident light angle of at least one of 0°, 15°, 30°, 45°, 60°, or 75°, at least 50, 60, 70, 80, 90, or 95 percent of incident ultraviolet light in a wavelength range from 195 nanometers or 200 nm, to 230 nanometers, 235 nm, or 240 nm, and collectively transmitting at an incident light angle of at least one of 0°, 15°, 30°, 45°, 60°, or 75°, at least 50, 60, 70, 80, 90, or 95 percent of incident ultraviolet light in a wavelength range from greater than 230 nanometers, greater than 235 nm, or greater than 240 nm, to 400 nanometers,

      wherein the broadband UVC light source is configured to direct light at the ultraviolet mirror of the multilayer article; and wherein the multilayer article is configured and arranged within the housing such that the absorbent layer absorbs ultraviolet light that is transmitted through the ultraviolet mirror to the absorbent layer

2. The device of claim 1, wherein the housing is configured to block direct passage of light from the broadband UVC light source through the window, with the exception of light that travels a distance of at least 3 centimeters (cm) from the broadband UVC light source to the window.

3. The device of claim 1, wherein the housing is configured to block direct passage of light from the broadband UVC light source through the window.

4. The device of any of claims 1 to 3, further comprising at least one angular control element disposed in the housing.

5. The device of claim 4, wherein the at least one angular control element comprises a concave shape.

6. The device of claim 5, wherein the broadband UVC light source is disposed between a first concave shape formed by the housing and the concave shape of the angular control element, wherein the first concave shape is curved in an opposing direction than the concave shape of the angular control element.

7. The device of any of claims 4 to 6, wherein the at least one angular control element comprises at least one of a collimator, a retroreflector, a diffuser, or a reflecting diverter.

8. The device of any of claims 4 to 7, wherein the at least one angular control element is disposed between the broadband UVC light source and the window.

9. The device of any of claims 1 to 8, wherein the absorbent layer comprises a silicone thermoplastic, a fluoropolymer, copolymers thereof, or blends thereof.

10. The device of any of claims 1 to 9, wherein the absorbent layer comprises a continuous metal coating or layer.

11. The device of any of claims 1 to 10, wherein the ultraviolet mirror absorbs at an incident light angle of at least one of 0°, 15°, 30°, 45°, 60°, or 75°, at least 30 percent, at least 80 percent, at least 90 percent, at least 95 percent, or at least 98 percent of incident visible light having a wavelength between at least 400 nanometers and 700 nanometers.

12. The device of any of claims 1 to 11, further comprising at least one of a heat transfer layer, a plurality of heat transfer fins, or a plurality of heat transfer pins, adjacent to a major surface of the absorbing layer opposite the ultraviolet mirror.

13. A method of disinfecting at least one material, the method comprising:

a) directing ultraviolet radiation emitted by the broadband UVC light source through the window of the device of any of claims 1 to 12; and
b) exposing the at least one material to the ultraviolet radiation passing through the window.

14. A system comprising: a device of any of claims 1 to 12; one or more electronic sensors configured to generate data that is indicative of an operation of the device; and at least one computing device comprising one or more computer processors and a memory comprising instructions that when executed by the one or more computer processors cause the one or more computer processors to: receive the data that is indicative of the operation of the device; apply the data to a disinfection model stored in the memory, the disinfection model characterizing the activity of the device; and wirelessly send to the device feedback that is based on the disinfection model, based on the identification of device activity.

15. A computing device comprising: one or more computer processors; and a memory comprising instructions that when executed by the one or more computer processors cause the one or more computer processors to: obtain usage data from a device of any of claims 1 to 12, wherein the usage data comprises data indicative of operation of the device; apply the usage data to a disinfection model stored in the memory, the disinfection model characterizing the activity of the device; and wirelessly send to the device feedback that is based on the disinfection model, based on the identification of device activity.

**Patentansprüche**

1. Eine Vorrichtung, aufweisend:

a) ein Gehäuse, das im Wesentlichen undurchlässig für Ultraviolettstrahlung, die eine Wellenlänge von 280 nm bis 400 nm hat, ist, und mindestens ein Fenster, das in dem Gehäuse definiert ist;
b) eine Breitband-UVC-Lichtquelle, die innerhalb des Gehäuses oder innerhalb einer optionalen Umhüllung angrenzend an das Gehäuse positioniert ist; und
c) einen mehrschichtigen Gegenstand, der innerhalb des Gehäuses positioniert ist, der mehrschichtige Gegenstand aufweisend:

i) eine absorbierende Schicht, die mindestens 50, 60, 70, 80, 90 oder 95 Prozent von einfallendem Ultraviolettlicht, das eine Wellenlänge zwischen mindestens 230 Nanometern und 400 Nanometern hat, absorbiert, die absorbierende Schicht aufweisend eine Hauptoberfläche; und
ii) einen Ultraviolettspiegel, der eine äußere Hauptoberfläche und eine innere Hauptoberfläche hat, wobei sich die innere Hauptoberfläche angrenzend an die Hauptoberfläche der absorbierenden Schicht befindet, wobei der Ultraviolettspiegel aus mindestens einer Mehrzahl von alternierenden ersten und zweiten optischen Schichten besteht, die gemeinsam, bei einem Einfallslichtwinkel von mindestens einem von 0°, 15°, 30°, 45°, 60° oder 75°, mindestens 50, 60, 70, 80, 90 oder 95 Prozent von einfallendem Ultraviolettlicht in einem Wellenlängenbereich von 195 Nanometern, oder 200 nm, bis 230 Nanometern, 235 nm oder 240 nm, reflektieren und gemeinsam, bei einem Einfallslichtwinkel von mindestens einem von 0°, 15°, 30°, 45°, 60° oder 75°, mindestens 50, 60, 70, 80, 90 oder 95 Prozent von einfallendem Ultraviolettlicht in einem Wellenlängenbereich von größer als 230 Nanometern, größer als 235 nm oder größer als 240 nm, bis 400 Nanometern transmittieren,

wobei die Breitband-UVC-Lichtquelle konfiguriert ist, um Licht auf den Ultraviolettspiegel des mehrschichtigen Gegenstands zu richten; und wobei der mehrschichtige Artikel konfiguriert und innerhalb des Gehäuse angeordnet ist, sodass die absorbierende Schicht Ultraviolettlicht absorbiert, das durch den Ultraviolettspiegel an die absorbierende Schicht transmittiert wird.

2. Die Vorrichtung nach Anspruch 1, wobei das Gehäuse konfiguriert ist, um einen direkten Durchgang von Licht von der Breitband-UVC-Lichtquelle durch das Fenster zu blockieren, mit der Ausnahme von Licht, das eine Entfernung von mindestens 3 Zentimetern (cm) von der Breitband-UVC-Lichtquelle zu dem Fenster zurücklegt.

3. Die Vorrichtung nach Anspruch 1, wobei das Gehäuse konfiguriert ist, um den direkten Durchgang von Licht von der Breitband-UVC-Lichtquelle durch das Fenster zu blockieren.

4. Die Vorrichtung nach einem der Ansprüche 1 bis 3, ferner aufweisend mindestens ein Winkelsteuerelement, das in dem Gehäuse eingerichtet ist.

5. Die Vorrichtung nach Anspruch 4, wobei das mindestens eine Winkelsteuerelement eine konkave Form aufweist.

6. Die Vorrichtung nach Anspruch 5, wobei die Breitband-UVC-Lichtquelle zwischen einer ersten konkaven Form, die mittels des Gehäuses ausgebildet wird, und der konkaven Form des Winkelsteuerelements eingerichtet ist, wobei die erste konkave Form in einer entgegengesetzten Richtung wie die konkave Form des Winkelsteuerelementes gekrümmt ist.

7. Die Vorrichtung nach einem der Ansprüche 4 bis 6, wobei das mindestens eine Winkelsteuerelement mindestens eines von einem Kollimator, einem Retroreflektor, einem Diffusor oder einem reflektierenden Umlenker aufweist.

8. Die Vorrichtung nach einem der Ansprüche 4 bis 7, wobei das mindestens eine Winkelsteuerelement zwischen der Breitband-UVC-Lichtquelle und dem Fenster eingerichtet ist.

9. Die Vorrichtung nach einem der Ansprüche 1 bis 8, wobei die absorbierende Schicht ein Silikonthermoplast, ein Fluorpolymer, Copolymere davon oder Mischungen davon aufweist.

10. Die Vorrichtung nach einem der Ansprüche 1 bis 9, wobei die absorbierende Schicht eine kontinuierliche Metallbeschichtung oder -schicht aufweist.

11. Die Vorrichtung nach einem der Ansprüche 1 bis 10, wobei der Ultraviolettspiegel, bei einem Einfallslichtwinkel von mindestens einem von 0°, 15°, 30°, 45°, 60° oder 75°, mindestens 30 Prozent, mindestens 80 Prozent, mindestens 90 Prozent, mindestens 95 Prozent oder mindestens 98 Prozent von einfallendem sichtbarem Licht, das eine Wellenlänge zwischen mindestens 400 Nanometern und 700 Nanometern hat, absorbiert.

12. Die Vorrichtung nach einem der Ansprüche 1 bis 11, ferner aufweisend mindestens eines von einer Wärmeübertragungsschicht, einer Mehrzahl von Wärmeübertragungsrippen oder einer Mehrzahl von Wärmeübertragungsstiften, angrenzend an eine Hauptoberfläche der absorbierenden Schicht gegenüberliegend dem Ultraviolettspiegel.

13. Ein Verfahren zum Desinfizieren mindestens eines Materials, das Verfahren aufweisend:

a) Richten von Ultraviolettstrahlung, die mittels der Breitband-UVC-Lichtquelle emittiert wird, durch das Fenster der Vorrichtung nach einem der Ansprüche 1 bis 12; und
b) Aussetzen des mindestens einen Materials gegenüber der Ultraviolettstrahlung, die durch das Fenster hindurchgeht.

14. Ein System, aufweisend: eine Vorrichtung nach einem der Ansprüche 1 bis 12; einen oder mehrere elektronische Sensoren, die konfiguriert sind, um Daten zu erzeugen, die einen Betrieb der Vorrichtung angeben; und mindestens eine Rechenvorrichtung, aufweisend einen oder mehrere Computerprozessoren und einen Speicher, aufweisend Anweisungen, die, wenn sie mittels des einen oder der mehreren Computerprozessoren ausgeführt werden, den einen oder die mehreren Computerprozessoren veranlassen zum: Empfangen der Daten, die den Betrieb der Vorrichtung angeben; Anwenden der Daten auf ein Desinfektionsmodell, das in dem Speicher gespeichert ist, wobei das Desinfektionsmodell die Aktivität der Vorrichtung kennzeichnet; und drahtloses Senden, an die Vorrichtung, einer Rückmeldung, die auf dem Desinfektionsmodell basiert, basierend auf der Identifizierung einer Vorrichtungsaktivität.

**15.** Eine Rechenvorrichtung, aufweisend: einen oder mehrere Computerprozessoren; und einen Speicher, aufweisend Anweisungen, die, wenn sie mittels des einen oder der mehreren Computerprozessoren ausgeführt werden, den einen oder die mehreren Computerprozessoren veranlassen zum: Erhalten von Nutzungsdaten von einer Vorrichtung nach einem der Ansprüche 1 bis 12, wobei die Nutzungsdaten Daten, die den Betrieb der Vorrichtung angeben, aufweisen; Anwenden der Nutzungsdaten auf ein Desinfektionsmodell, das in dem Speicher gespeichert ist, wobei das Desinfektionsmodell die Aktivität der Vorrichtung kennzeichnet; und drahtloses Senden, an die Vorrichtung, der Rückmeldung, die auf dem Desinfektionsmodell basiert, basierend auf der Identifizierung der Vorrichtungsaktivität.

**Revendications**

1. Dispositif comprenant :

   a) un boîtier sensiblement imperméable aux rayonnements ultraviolets ayant une longueur d'onde allant de 280 nm à 400 nm, et au moins une fenêtre définie dans le boîtier ;
   b) une source de lumière UVC à large bande positionnée à l'intérieur du boîtier ou à l'intérieur d'une enceinte facultative adjacente au boîtier ; et
   c) un article multicouche positionné à l'intérieur du boîtier, l'article multicouche comprenant :

      i) une couche absorbante qui absorbe au moins 50, 60, 70, 80, 90 ou 95 pour cent de la lumière ultraviolette incidente ayant une longueur d'onde comprise entre au moins 230 nanomètres et 400 nanomètres, la couche absorbante comprenant une surface principale ; et
      ii) un miroir ultraviolet ayant une surface principale externe et une surface principale interne, la surface principale interne étant adjacente à la surface principale de la couche absorbante, dans lequel le miroir ultraviolet est constitué d'au moins une pluralité de premières et secondes couches optiques alternées réfléchissant collectivement à un angle de lumière incidente d'au moins l'un parmi 0°, 15°, 30°, 45°, 60°, ou 75°, au moins 50, 60, 70, 80, 90, ou 95 pour cent de la lumière ultraviolette incidente dans une gamme de longueurs d'onde de 195 nanomètres ou 200 nm à 230 nanomètres, 235 nm, ou 240 nm et transmettant collectivement à un angle de lumière incidente d'au moins l'un parmi 0°, 15°, 30°, 45°, 60°, ou 75°, au moins 50, 60, 70, 80, 90, ou 95 pour cent de la lumière ultraviolette incidente dans une gamme de longueurs d'onde allant de plus de 230 nanomètres, plus de 235 nm ou plus de 240 nm à 400 nanomètres, dans lequel la source de lumière UVC à large bande est configurée pour diriger la lumière au niveau du miroir ultraviolet de l'article multicouche ; et dans lequel l'article multicouche est conçu et disposé à l'intérieur du boîtier de telle sorte que la couche absorbante absorbe la lumière ultraviolette qui est transmise à travers le miroir ultraviolet à la couche absorbante.

2. Dispositif selon la revendication 1, dans lequel le boîtier est conçu pour bloquer le passage direct de la lumière provenant de la source de lumière UVC à large bande à travers la fenêtre, à l'exception de la lumière qui parcourt une distance d'au moins 3 centimètres (cm) de la source de lumière UVC à large bande à la fenêtre.

3. Dispositif selon la revendication 1, dans lequel le boîtier est conçu pour bloquer le passage direct de la lumière provenant de la source de lumière UVC à large bande à travers la fenêtre.

4. Dispositif selon l'une quelconque des revendications 1 à 3, comprenant en outre au moins un élément de commande angulaire disposé dans le boîtier.

5. Dispositif selon la revendication 4, dans lequel l'au moins un élément de commande angulaire comprend une forme concave.

6. Dispositif selon la revendication 5, dans lequel la source de lumière UVC à large bande est disposée entre une première forme concave formée par le boîtier et la forme concave de l'élément de commande angulaire, dans lequel la première forme concave est incurvée dans une direction opposée à la forme concave de l'élément de commande angulaire.

**7.** Dispositif selon l'une quelconque des revendications 4 à 6, dans lequel l'au moins un élément de commande angulaire comprend au moins l'un parmi un collimateur, un rétroréflecteur, un diffuseur, ou un inverseur réfléchissant.

**8.** Dispositif selon l'une quelconque des revendications 4 à 7, dans lequel l'au moins un élément de commande angulaire est disposé entre la source de lumière UVC à large bande et la fenêtre.

**9.** Dispositif selon l'une quelconque des revendications 1 à 8, dans lequel la couche absorbante comprend un thermoplastique de silicone, un fluoropolymère, des copolymères de ceux-ci, ou des mélanges de ceux-ci.

**10.** Dispositif selon l'une quelconque des revendications 1 à 9, dans lequel la couche absorbante comprend un revêtement ou une couche métalliques continus.

**11.** Dispositif selon l'une quelconque des revendications 1 à 10, dans lequel le miroir ultraviolet absorbe à un angle de lumière incidente d'au moins l'un parmi 0°, 15°, 30°, 45°, 60° ou 75°, au moins 30 pour cent, au moins 80 pour cent, au moins 90 pour cent, au moins 95 pour cent, ou au moins 98 pour cent de lumière visible incidente ayant une longueur d'onde comprise entre au moins 400 nanomètres et 700 nanomètres.

**12.** Dispositif selon l'une quelconque des revendications 1 à 11, comprenant en outre au moins l'une parmi une couche de transfert de chaleur, une pluralité d'ailettes de transfert de chaleur, ou une pluralité de broches de transfert de chaleur, adjacente à une surface principale de la couche absorbante opposée au miroir ultraviolet.

**13.** Procédé de désinfection d'au moins un matériau, le procédé comprenant :

a) le fait de diriger un rayonnement ultraviolet émis par la source de lumière UVC à large bande à travers la fenêtre du dispositif selon l'une quelconque des revendications 1 à 12 ; et
b) l'exposition de l'au moins un matériau au rayonnement ultraviolet passant à travers la fenêtre.

**14.** Système comprenant un dispositif selon l'une quelconque des revendications 1 à 12 ; un ou plusieurs capteurs électroniques configurés pour générer des données qui indiquent un fonctionnement du dispositif ; et au moins un dispositif informatique comprenant un ou plusieurs processeurs informatiques et une mémoire comprenant des instructions qui, lorsqu'elles sont exécutées par le ou les processeurs informatiques, amènent le ou les processeurs informatiques à : recevoir les données qui indiquent le fonctionnement du dispositif ; appliquer les données à un modèle de désinfection stocké dans la mémoire, le modèle de désinfection caractérisant l'activité du dispositif ; et envoyer sans fil au dispositif une rétroaction d'information qui est basée sur le modèle de désinfection, sur la base de l'identification de l'activité du dispositif.

**15.** Dispositif informatique comprenant : un ou plusieurs processeurs informatiques ; et une mémoire comprenant des instructions qui, lorsqu'elles sont exécutées par le ou les processeurs informatiques, amènent le ou les processeurs informatiques à : obtenir des données d'utilisation à partir d'un dispositif selon l'une quelconque des revendications 1 à 12, dans lequel les données d'utilisation comprennent des données indiquant un fonctionnement du dispositif ; appliquer les données d'utilisation à un modèle de désinfection stocké dans la mémoire, le modèle de désinfection caractérisant l'activité du dispositif ; et envoyer sans fil au dispositif une rétroaction d'information qui est basée sur le modèle de désinfection, sur la base de l'identification de l'activité du dispositif.

100 ⟶

19

12A
13A
12B
13B

5

12N
13N

11

14
18

15

*FIG. 1A*

100 ⟶

19

12A
13A
12B
13B

5

12N
13N

11

14
18
16
17

4

*FIG. 1B*

Example 1

*FIG. 2*

300

306

302

312

310

304

308

314

316

*FIG. 3*

*FIG. 4A*

*FIG. 4B*

*FIG. 5A*

*FIG. 5B*

System Efficiency

Efficiency

1.0 0.9 0.8 0.7 0.6 0.5 0.4 0.3 0.2 0.1 0.0

10 15 20 25 30 35 40 45 50 55 60 65 70 75 80 85 90 95 100

Mirror Reflectivity

*FIG. 5C*

*FIG. 6A*

*FIG. 6B*

*FIG. 6C*

*FIG. 6D*

EP 4 362 990 B1

704    702    700

706

708

*FIG. 7A*

704    702

700

714

*FIG. 7B*

FIG. 7C

*FIG. 7D*

FIG. 8B

FIG. 8A

FIG. 8C

*FIG. 8D*

Obtain a device according to the first aspect and directing ultraviolet radiation emitted
by the broadband UVC light source through the window
910

Expose the at least one material to the ultraviolet radiation passing through the window for
a time sufficient to achieve a desired degree of disinfection of the at least one material
920

Optionally, expose until achievement of a log 2, log 3, log 4, or greater reduction in an
amount of at least one microorganism present on or in the at least one material, as
compared to an amount of the at least one microorganism present prior to exposing the at
least one material to the ultraviolet radiation passing through the window
930

*FIG. 9*

Environment
8A

Network
4

Equipment Management System
6

2

Environment
8B

7

26

Equipment Stations
21

19A

19B

23C

25

User 20A ... User 20N

10A

10B

10N

23A

23B

Remote User 24A ... Remote User 24N

FIG. 10

EP 4 362 990 B1

## Equipment Management System
### 6

| Event Data 74A | Historical Data and Models 74B | Audit Data 74C | Config Data 74D | Work Relat. Data 74E |
|---|---|---|---|---|

72

| Event Endpoint Frontend 68A | Event Selector 68B | Event Processor 68C | HP Event Processor 68D |
|---|---|---|---|
| Notification Service 68E | Stream Analytics Service 68F | Record MGMT and Reporting Service 68G | Security Service 68H |

68

## Operating System
### 36

70

| Memory 32 | Processors 28 | I/O Interface 34 |
|---|---|---|

60

75A

75B

75C

62

21

10A

10B    10N

Equipment Stations

*FIG. 11*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020070589 A1
- US 5882774 A, Jonza
- US 6045894 A, Jonza
- US 6368699 B, Gilbert
- US 6531230 B, Weber
- US 6667095 B, Wheatley
- US 6783349 B, Neavin
- US 7271951 B2, Weber
- US 7632568 B2, Padiyath
- US 7652736 B2, Padiyath
- US 7952805 B2, McGurran
- WO 9517303 A, Ouderkirk
- WO 9939224 A, Ouderkirk
- WO 2019130198 A, Hebrink
- US 5504134 A, Palmer
- US 5876688 A, Laundon
- EP 521553 A, N. V. Philips
- DE 4302555 A1

### Non-patent literature cited in the description

- Standard Practice for Measuring and Compensating for Emissivity Using Infrared Imaging Radiometers. *ASTM E1933-14*, 2018
- **PARKYN et al.** The Black HoleTM: Cuspated waveguide-injectors and illuminators for LEDs. *Proc. SPIE 3781, Nonimaging Optics: Maximum Efficiency Light Transfer V*, 06 October 1999
- **KOGELSCHATZ**. *Applied Surface Science*, 1992, vol. 54, 410-423
- **KITAMURA et al.** *Applied Surface Science*, 1994, vol. 79/80, 507-513
- *Tech. Phys*, 1994, vol. 39 (10), 1054